# EUROPEAN PATENT APPLICATION

(11) **EP 3 144 877 A1**
(43) Date of publication of application: **22.03.2017**
(21) Application number: 15792488.7
(22) Date of filing: 12.05.2015
(51) Int. Cl.: G06Q 50/24, A61H 39/02, G06Q 50/22

(54) **ACUPUNCTURE AND MOXIBUSTION TREATMENT SUPPORT TERMINAL DEVICE AND MEDICAL SUPPORT SYSTEM**

(30) Priority: 12.05.2014 JP 2014098651
(71) Applicant: Oda Satoshi, Tokyo 102-0093 (JP)
(72) Inventor: Oda Satoshi, Tokyo 102-0093 (JP)
(74) Representative: Heine, Christian Klaus
(86) International application number: PCT/JP2015/063674
(87) International publication number: WO 2015/174425

(57) **Abstract**

To provide an acupuncture and moxibustion treatment support terminal device and a medical support system that ensures to manage data on a patient's symptoms, contents of a treatment, and the like in a clinic, to properly supply information on acupoints and the like to an acupuncturist, and to build a network among clinics, hospitals, and patients. The acupuncture and moxibustion treatment support terminal device is a terminal device 100 provided with a display input unit adapted to display information from and input information to a terminal or a server that stores two-dimensional or three-dimensional information on a human body model 35a in which marks locating human acupoints and names and/or codes of the acupoints are recorded. The display input unit displays an image of the human body model on which the marks locating human acupoints and names and/or codes of the acupoints are displayed superimposed at corresponding locations thereof.

## Description

### TECHNICAL FIELD

The present invention relates to an acupuncture and moxibustion treatment support terminal device and medical support system that support acupuncture and moxibustion treatment.

### BACKGROUND ART

An acupuncture and moxibustion treatment, generally called "harikyuu" or "shinkyuu" in Japan, is a Japanese traditional medicine and widely practiced as a branch of Oriental Medicine. The acupuncture and moxibustion treatment is a treatment technique such that an acupuncture point (hereinafter, referred to as an "acupoint") is inserted with a thin metal needle, or moxa is burned on the acupoint, thereby stimulating the acupoint. The acupuncture and moxibustion treatment is performed by selecting an acupoint according to symptoms and constitution, and then applying the stimulation with the needle and/or moxa to the acupoint. However, it requires an expert knowledge and much experience to select a location of the acupoint.

Furthermore, it is a general practice for clinics that perform the acupuncture and moxibustion treatment to preserve medical charts of manipulative therapies such as massage and the acupuncture and moxibustion treatment by filling a paper-printed form using a writing tool such as a ballpoint pen. It is also a common practice that patients fill in check boxes and blanks of a questionnaire and an interview sheet printed on paper using a ballpoint pen or the like.

Furthermore, it is the current situation that each clinic independently selects locations of patient's acupoints, and information thereof is not exchanged among clinics and between clinics and hospitals.

Incidentally, for example, Japanese Unexamined Patent Application, Publication No. 2006-61256 (Patent Document 1) discloses a technique related to an acupuncture and moxibustion diagnostic system that enables even a nonskilled person to easily determine a treatment method and locations of acupoints.
Patent Document 1: Japanese Unexamined Patent Application, Publication No. 2006-61256

### DISCLOSURE OF THE INVENTION

### Problems to be Solved by the Invention

In view of promoting integrated medicine in Japan, cooperation of medical institutions and complementary and alternative medical institutions is required. For the sake of the cooperation, it is necessary for the complementary and alternative medical institutions to record the contents of treatments. Presently, however, there is no unified format of the medical charts, and insufficient education is provided for creating the medical charts. In particular, names of the acupoints to be used in the acupuncture and moxibustion treatment, and Shiatsu massage are expressed in Chinese characters, which is hard to describe. Owing to recent reports of World Health Organization (herein referred to as "WHO"), the names of the acupoints are internationally unified. However, conventional names are still used in many cases. This fact not only makes it difficult for the medical institutions and complementary and alternative medical institutions to cooperate with each other, but also acts as a major barrier to the unification of the treatments and the standardization of evidence buildings.

The present invention has been made to solve the above-mentioned problems, and it is an object of the present invention to provide an acupuncture and moxibustion treatment support terminal device and a medical support system that can reliably manage data of symptoms of patients, contents of the treatment, and the like in a clinic, accurately provide an acupuncturist with information on the acupoints and the like, and/or construct networks among clinics, hospitals, and patients.

### Means for Solving the Problems

The present invention has the following configuration for solving the above-mentioned problems.

In accordance with a first aspect of the present invention, there is provided an acupuncture and moxibustion treatment support terminal device, including: a display input unit adapted to display information from and input information to a terminal or a server that stores two-dimensional or three-dimensional information on a human body model in which marks locating human acupoints and names and/or codes of the acupoints are recorded, wherein the display input unit displays a figure of the human body model on which the marks locating human acupoints and names and/or codes of the acupoints are displayed superimposed at corresponding locations thereof.

In accordance with a second aspect of the present invention, the display input unit displays at least one list of the names and/or the codes of the acupoints in place of the marks locating human acupoints and the names and/or the codes of the acupoints superimposed at the corresponding locations thereof.

In accordance with a third aspect of the present invention, the display input unit displays an image of an input tool for treatment, the input tool including an input button or an input slider, and, by operating the input button or the input slider, the contents of the treatment are stored and displayed on the display input unit along with the marks locating human acupoints and the names and/or the codes of the acupoints.

In accordance with a fourth aspect of the present invention, the acupuncture and moxibustion treatment support terminal device further includes an image capture unit for capturing an image, the display input unit displays an image acquisition button for causing the image capture unit to start image capturing, and, by operating the image acquisition button, the image captured by the image capture unit is stored and the stored image is displayed on the display input unit along with the marks locating human acupoints and the names and/or the codes of the acupoints.

In accordance with a fifth aspect of the present invention, the figure of the two-dimensional or three-dimensional human body model displayed on the display input unit can be enlarged, shrunk, rotated, or moved by operating with a finger a screen of the display input unit.

In accordance with a sixth aspect of the present invention, the acupuncture and moxibustion treatment support terminal device stores a plurality of figures each showing a part of the human body, the display input unit displays the figures and a plurality of buttons for selecting symptoms, and, after a predetermined figure is selected from among the figures, an affected area is inputted in and displayed on the display input unit by selecting one of the buttons, and drawing the affected area with a finger on the predetermined figure.

In accordance with a seventh aspect of the present invention, there is provided an acupuncture and moxibustion treatment support terminal device, including: a display input unit adapted to display information from and input information to a terminal or a server that stores information on a plurality of figures each showing a part of the human body, wherein the display input unit displays the figures and a plurality of buttons for selecting symptoms, and, after a predetermined figure is selected from among the figures, an affected area is inputted in and displayed on the display input unit by selecting one of the buttons and drawing the affected area with a finger on the predetermined figure.

In accordance with an eighth aspect of the present invention, the display input unit displays a field for inputting a comment.

In accordance with a ninth aspect of the present invention, the acupuncture and moxibustion treatment support terminal device stores a plurality of predefined texts, and a comment selected from among the predefined texts is inputted in the field.

In accordance with a tenth aspect of the present invention, the acupuncture and moxibustion treatment support terminal device can display a selection button for selecting and inputting an oriental medical opinion on a patient, a selection button for selecting and inputting information on a color and a shape related to the oriental medical opinion, and a field having a comment on the oriental medical opinion.

In accordance with an eleventh aspect of the present invention, the display input unit displays a human full body shape and a plurality of buttons for selecting a treatment for a predetermined human body area, and, by selecting one of the buttons to select the treatment and drawing the human full body shape, a location where the selected treatment has been performed is inputted in and displayed on the display input unit.

In accordance with a twelfth aspect of the present invention, the display input unit displays a plurality of buttons for selecting a treatment for another human body area different from that for the predetermined human body area, sliders for inputting duration and amounts of a treatment, and a field for inputting a comment on the treatment, the treatment for the another human body area and duration and amounts of the treatment are inputted in and displayed on the display input unit by operating the buttons for selecting the treatment for another human body area and operating the slider with a finger, and the comment is inputted by inputting in the field or by selecting one of the predefined texts.

In accordance with a thirteenth aspect of the present invention, the display input unit displays an interview sheet, and, by filling in items of the interview sheet, the items in the interview sheet are inputted in and displayed on the display input unit and are stored in the acupuncture and moxibustion treatment support terminal device.

In accordance with a fourteenth aspect of the present invention, in a case in which one of the items of the interview sheet is to be inputted, one of a plurality of predefined texts or one of a plurality of displayed buttons is selected to input the item.

In accordance with a fifteenth aspect of the present invention, the display input unit displays an image of a product and a button for submitting a purchase order of the product.

In accordance with a sixteenth aspect of the present invention, the acupuncture and moxibustion treatment support terminal device further includes; a reservation reception unit for accepting a reservation of a patient, wherein the acupuncture and moxibustion treatment support terminal device stores electronic medical charts on a plurality of patients and a reservation calendar in which appointments of the patients accepted by the reservation reception unit are inputted, wherein by means of the display input unit, the reservation calendar is displayed, and the appointments of the patients accepted by the reservation reception unit are inputted in the reservation calendar.

In accordance with a seventeenth aspect of the present invention, the acupuncture and moxibustion treatment support terminal device accepts a reservation of a new patient who has not yet been registered, assigns a specific time zone as a reserved time from an available time zone in the reservation calendar, accepts an input of information on the electronic medical charts on the new patient, and causes the display input unit to display the reserved time and the information on the electronic medical charts in association with each other.

In accordance with an eighteenth aspect of the present invention, there is provided a medical support system in which at least one clinic that performs a complementary and alternative medicine, a medical center at which a doctor is enrolled, and a home of a patient to receive the complementary and alternative medicine are connected to a server via a network, wherein the clinic is provided with the acupuncture and moxibustion treatment support terminal device according to any one of the first to seventeenth aspects of the present invention, the medical center is provided with a personal computer or the acupuncture and moxibustion treatment support terminal device according to any one of the first to seventeenth aspects of the present invention, the acupuncture and moxibustion treatment support terminal device at the clinic and the personal computer or the acupuncture and moxibustion treatment support terminal device at the medical center exchange the information on the electronic medical charts of the patient with each other via the network, and the patient makes a reservation with the clinic via the network.

In accordance with a nineteenth aspect of the present invention, the at least one clinic that performs the complementary and alternative medicine includes a plurality of clinics, and acupuncturists at respective clinics exchange with one another information including the information on the electronic medical charts on the patient via the network.

In accordance with a twentieth aspect of the present invention, the information on the electronic medical charts on the patient exchanged via the network is outputted to existing electronic medical charts stored in a medical institution.

In accordance with a twenty first aspect of the present invention, the acupuncture and moxibustion treatment support terminal device provided at the clinic displays an advertisement for the patient's reference via the network or a screen for the patient to purchase a product.

### Effects of the Invention

According to the present invention, it is possible to provide an acupuncture and moxibustion treatment support terminal device and a medical support system that can reliably manage data indicative of symptoms of a patient, contents of treatment, and the like in a clinic, can accurately inform an acupuncturist of the acupoints and the like, and further can construct a network of clinics, hospitals, and patients.

### BRIEF DESCRIPTION OF THE DRAWINGS

Fig. 1 is a diagram showing a usage state of a tablet terminal comprising an acupuncture and moxibustion treatment support terminal device according to a first embodiment;
Fig. 2A is a diagram showing a data structure of data regions stored in the acupuncture and moxibustion treatment support terminal device according to the first embodiment;
Fig. 2B is a diagram showing a hierarchic structure of privileges to login the data regions;
Figs. 3A and 3B are diagrams illustrating a paint tool of the acupuncture and moxibustion treatment support terminal device according to the first embodiment;
Fig. 4 is a diagram illustrating a button tool of the acupuncture and moxibustion treatment support terminal device according to the first embodiment;
Figs. 5A and 5B are diagrams illustrating a slider tool of the acupuncture and moxibustion treatment support terminal device according to a first embodiment;
Fig. 6 is a diagram illustrating a comment module of the acupuncture and moxibustion treatment support terminal device according to the first embodiment;
Figs. 7A to 7C are diagrams showing screens when starting operation of the acupuncture and moxibustion treatment support terminal device according to the first embodiment;
Figs. 8A to 8E are diagrams showing screens of a reception region and the like of the acupuncture and moxibustion treatment support terminal device according to the first embodiment;
Figs. 9A to 9C are diagrams showing screens of a patient medical chart and the like of the acupuncture and moxibustion treatment support terminal device according to the first embodiment;
Figs. 10A and 10B are diagrams showing screens of a questionnaire region of the acupuncture and moxibustion treatment support terminal device according to the first embodiment;
Figs. 11A to 11F are diagrams showing screens of a subjective assessment region of the acupuncture and moxibustion treatment support terminal device according to the first embodiment;
Fig. 12 is a diagram showing a screen of an objective finding region of the acupuncture and moxibustion treatment support terminal device according to the first embodiment;
Figs. 13A to 13C are diagrams showing other screens of the objective finding region of the acupuncture and moxibustion treatment support terminal device according to the first embodiment;
Figs. 14A to 14D are diagrams showing screens of a tongue diagnosis region of the acupuncture and moxibustion treatment support terminal device according to the first embodiment;
Figs. 15A and 15B are diagrams showing screens of a pulse diagnosis region of the acupuncture and moxibustion treatment support terminal device according to the first embodiment;
Figs. 16A to 16C are diagrams showing screens of an abdominal diagnosis region of the acupuncture and moxibustion treatment support terminal device according to the first embodiment;
Figs. 17A and 17B are first diagrams showing a screen of an acupoint treatment region of the acupuncture and moxibustion treatment support terminal device according to the first embodiment;
Fig. 18 is a second diagram showing another screen of the acupoint treatment region of the acupuncture and moxibustion treatment support terminal device according to the first embodiment;
Figs. 19A to 19C are third diagrams showing still other screens of the acupoints treatment region of the acupuncture and moxibustion treatment support terminal device according to the first embodiment;
Figs. 20A to 20C are fourth diagrams showing further still other screens of the acupoint treatment region of the acupuncture and moxibustion treatment support terminal device according to the first embodiment;
Figs. 21A to 21D are diagrams showing screens of an area treatment region of the acupuncture and moxibustion treatment support terminal device according to the first embodiment;
Fig. 22 is a diagram showing a screen of a treatment evaluation region of the acupuncture and moxibustion treatment support terminal device according to the first embodiment;
Fig. 23 is a diagram showing a screen for product purchase displayed by the acupuncture and moxibustion treatment support terminal device according to a fourth embodiment; and
Fig. 24 is a diagram showing a network of a medical support system according to a fifth embodiment.

### PREFERRED MODE FOR CARRYING OUT THE INVENTION

In the following, a description will be given of embodiments of the present invention with reference to drawings.

### <First Embodiment>

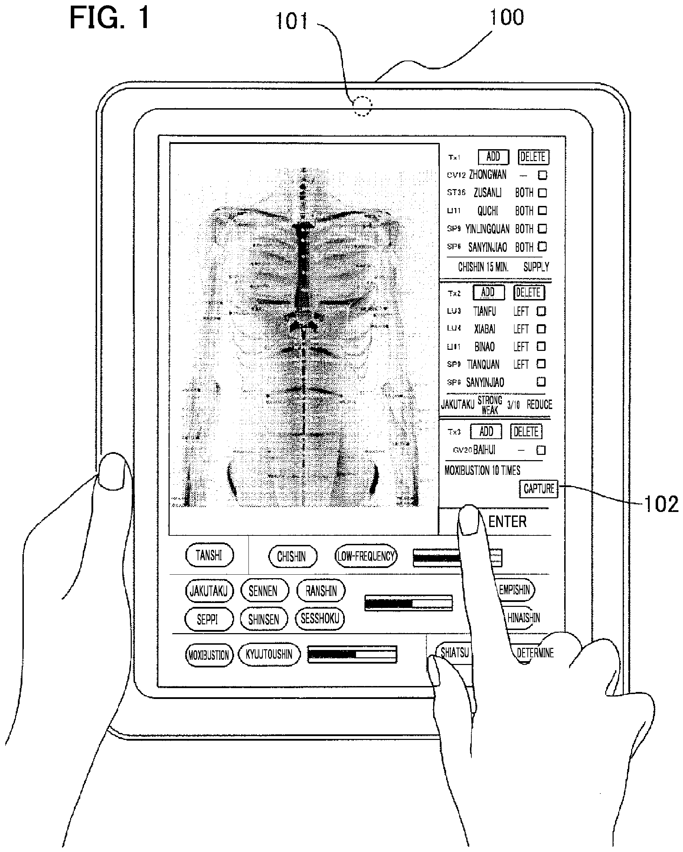

The acupuncture and moxibustion treatment support terminal device and the medical support system according to a first embodiment is implemented based on creating data of electronic medical charts for each patient using a tablet terminal 100 shown in Fig. 1. In place of the tablet terminal 100, a personal computer, a cell phone, or the like may be applicable. An acupuncturist can interactively use the tablet terminal 100, and contents of the interaction are recorded in a cloud (a remote server). Furthermore, a patient can use the tablet terminal 100 to input items of an interview sheet for the patient to fill in. The tablet terminal 100 is adapted to change the color of an image displayed thereon and to make a sound while a user operates the tablet terminal 100 to input therein, to ensure that a user can confirm the input. The tablet terminal 100 is provided with a memory as a storage unit for storing information on the electronic medical charts, a display screen (display input unit) for allowing a user to input predetermined information while displaying the information on the electronic medical charts, and a communication unit for communicating with outside devices. The tablet terminal 100 is further provided with a camera 101 (an image capture unit) for capturing an image of an affected area and the like of the patient, and a capture button 102 (an image acquisition button) to cause the camera 101 to start image capturing.

### <Configuration of Electronic Medical Charts of Acupuncture and Moxibustion Treatment Support Terminal Device>

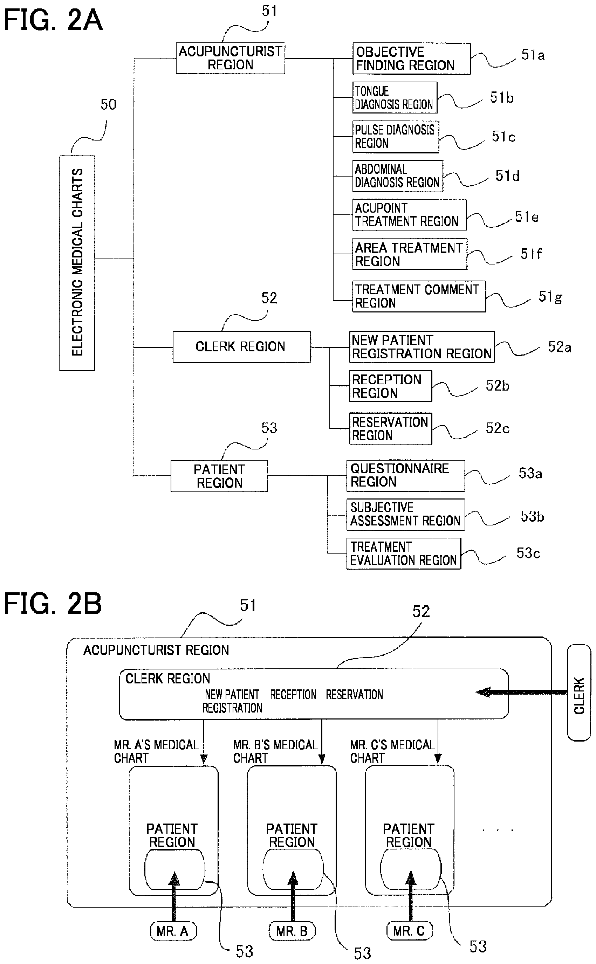

As shown in Fig. 2A, an acupuncturist region 51, a clerk region 52, and a patient region 53, respectively allowing an acupuncturist, a clerk, and a patient to view and input, are allocated to electronic medical charts 50 stored in the acupuncture and moxibustion treatment support terminal device according to the present embodiment. The acupuncturist region 51 is constituted of an objective finding region 51a, a tongue diagnosis region 51b, a pulse diagnosis region 51c, an abdominal diagnosis region 51d, an acupoint treatment region 51e, an area treatment region 51f, and a treatment comment region 51g. The clerk region 52 is constituted of a new patient registration region 52a, a reception region 52b, and a reservation region 52c. The patient region 53 is constituted of a questionnaire region 53a, a subjective assessment region 53b, and a treatment evaluation region 53c.

Fig. 2B shows a relationship among the acupuncturist region 51, the clerk region 52, and the patient region 53. The acupuncturist region 51 encloses the clerk region 52, and patient medical charts. Each patient medical chart includes a patient region 53 for each patient. In order to access to each of the patient regions 53, an access right is required. Similarly, in order to log out of the patient region 53, a log-out right is required. A clerk can log-in to the clerk region 52 and use the new patient registration region 52a, the reception region 52b, and the reservation region 52c. Before and/or after a treatment, the tablet terminal 100 is handed to the patient in a state in which the tablet terminal 100 is locked up to ensure that the patient can only utilize the patient region 53 of his/her own medical chart, and the patient inputs items of "Questionnaire", "Subjective Assessment", and "Treatment Evaluation". Hereinafter, descriptions will be given of how the acupuncturist, the clerk, and the patient can access to the respective regions.

### (1) Acupuncturist

Each acupuncturist is assigned with his/her own access account. Using an ID and a password of the account, each acupuncturist logs in to the electronic medical charts 50. The acupuncturist account allows the acupuncturist to access the above described three of the acupuncturist region 51, the clerk region 52, and the patient region 53, which are provided as the electronic medical charts 50.

### (2) Clerk

The acupuncturist can create a child account and assign the child account to each of his/her clerks. The clerk account allows the clerk to access the new patient registration region 52a, the reception region 52b, and the reservation region 52c.

### (3) Patient

The acupuncturist hands to the patient the tablet terminal 100 which constitutes the acupuncture and moxibustion treatment support terminal device, in a state that the tablet terminal 100 is locked up to ensure that the patient can only utilize the patient region 53 of his/her own medical chart. The patient can only input the items of "Questionnaire", "Subjective Assessment", and "Treatment Evaluation". Even if the input is complete, the patient cannot exit the patient region 53 without authorization.

The screens of the acupuncturist region 51, the clerk region 52, and the patient region 53 have a medical chart module and a comment module as common modules. In the following, a detailed description will be given of the medical chart module and the comment module.

### (1) Medical chart Module

The medical chart module is constituted of a paint tool, a button tool, a slider tool, and a text tool. The acupuncturist, the clerk, and the patient can input a "location" by means of the paint tool, select and input an "item" by means of the button tool, input a "numeric value" by means of the slider tool, and input a "text" by means of the text tool.

### (a) Paint Tool

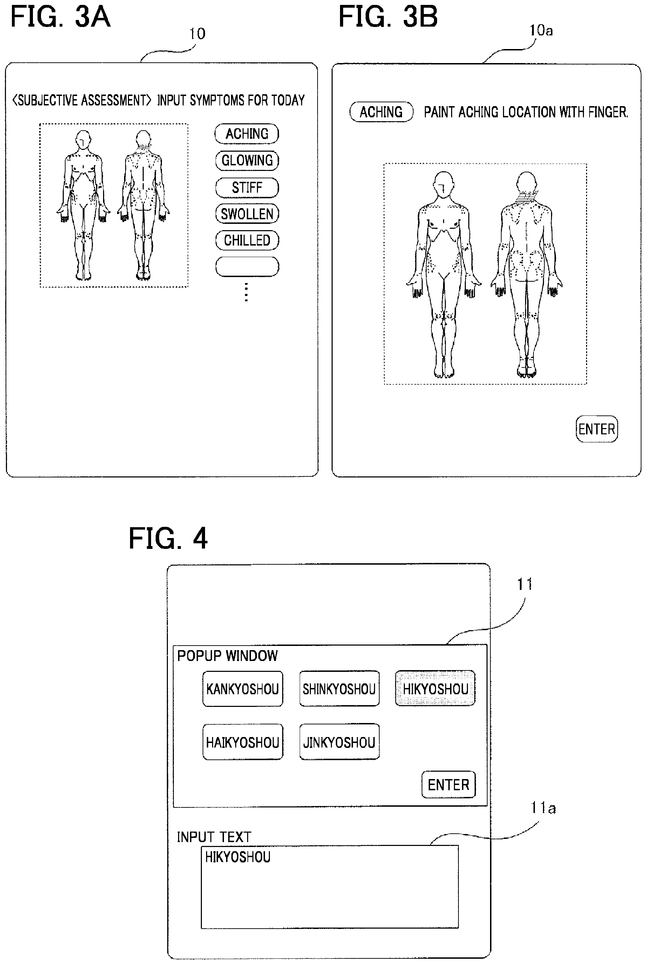

The paint tool serves as a tool for designating a "location" by painting with a finger on a schema (a figure showing a human body) on a screen of the tablet terminal 100. For example, in a case in which the back of the head aches, the patient taps an <Aching> button (Fig. 3A) on a screen 10 displayed by the medical chart module on the tablet terminal 100 to move to a paint input screen 10a (Fig. 3B), and paints the back of the head with a finger. Thus, it is possible to specify that the back of the head is "aching".

### (b) Button Tool

The button tool serves as a tool for inputting an option by tapping one of option buttons displayed on the screen of the tablet terminal 100 as shown in Fig. 4. For example, it is possible to input a text "Hikyosho" in a predetermined input field 11a in the medical chart module by tapping a <Hikyosho> button shown in a popup window 11 and tapping an <Enter> button.

### (c) Slider Tool

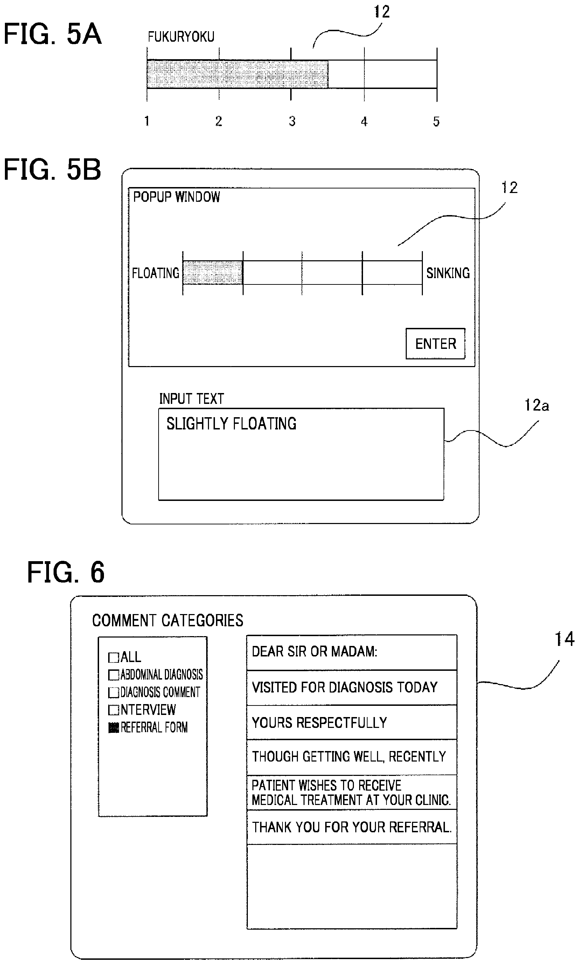

The slider tool serves as a tool for mainly inputting a numeric value as shown in Fig. 5A. For example, in a case in which Fukuryoku (tension of the abdominal wall) is evaluated to be 3.5 on a scale of one to five, it is possible to input a numerical number by running a finger over a slider 12 on the screen of the tablet terminal 100. The slider 12 can input a step value incremented by a designated value (such as 0.5, 1.0, or the like) most close to a position of the slider designated by the finger. Furthermore, the slider tool may be employed to select and input a text in place of the numeric value. For example, in the pulse diagnosis, a state of the pulse is expressed by texts in five levels such as "floating", "slightly floating", "middle", "slightly sinking", and "sinking", as a practice of acupuncture and moxibustion medicine. In a case of inputting the text "slightly floating", by sliding the slider 12 from a central position slightly toward a side of "floating" and tapping an <Enter> button (Fig. 5B), it is possible to input the text "slightly floating" in a predetermined text input field 12a in the medical chart module.

### (d) Text Tool

The text tool serves as a tool for freely inputting a text in a text input field by a keyboard. In the text tool, it is always possible to call a comment module described below, thereby easily inserting a predefined text that has been inputted in advance.

### (2) Comment Module

The comment module allows a user to register in advance, as predefined texts, frequently used sentences, words, and the like that are not yet selectable in the comments by the button tools. In this manner, when a text is inputted, a user can activate and invoke the comment module and insert one or more of the predefined texts in a text input field. As shown in a screen 14 (Fig. 6) of the tablet terminal 100, the predefined texts may be classified into "Comment Categories" according to respective purposes of usage. When one of the comment categories is selected, the predefined texts in the selected category are invoked and displayed in a ranking order so that more appropriate input candidates are listed higher. Upon selecting and tapping one of the displayed predefined texts, the selected predefined text is inserted in a text input field.

### <Starting Operation of Acupuncture and Moxibustion Treatment Support Terminal Device>

In the following, a sequential description will be given of an example in a case in which an acupuncturist starts operation of the acupuncture and moxibustion treatment support terminal device according to the present embodiment.

### (1) Login

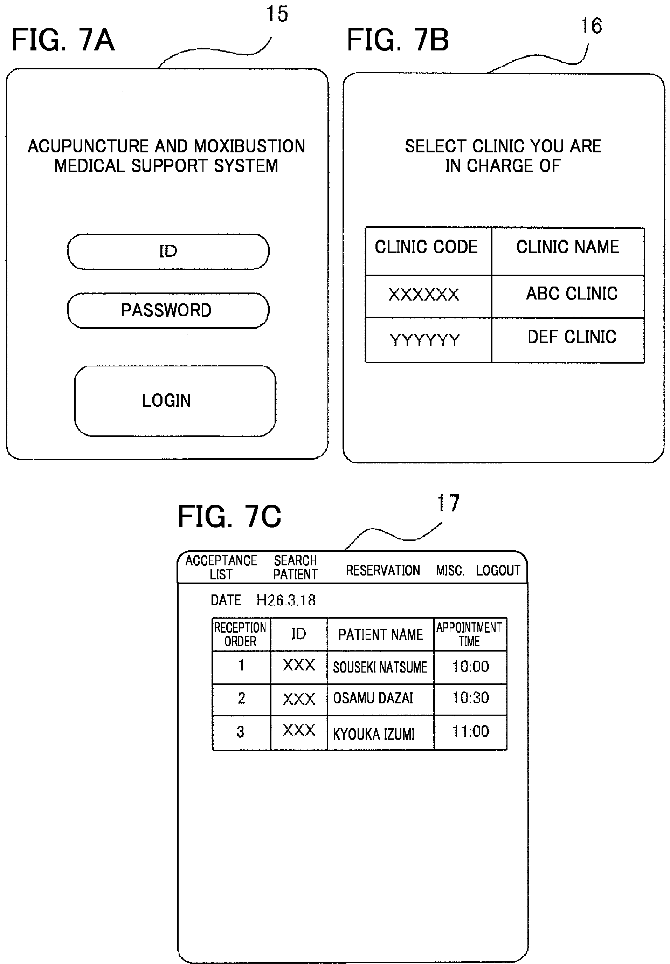

In a screen 15 of the tablet terminal 100 of Fig. 7A, the acupuncturist logs in using the ID and the password assigned at a time of contract.

### (2) Clinic Selection

In a case in which the acupuncturist manages a plurality of clinics by him/herself, the acupuncturist selects one of the clinics to take charge of the treatment in a screen 16 (Fig. 7B) of the tablet terminal 100.

### (3) Acceptance List (Reception Region)

When the acupuncturist selects the clinic, a list of accepted patients is displayed as shown on a screen 17 (Fig. 7C) of the tablet terminal 100. It is possible to display the list of accepted patients at any time by tapping "Acceptance List" on a menu bar. When the acupuncturist taps a "Name" of the patient, the patient medical chart for the patient is invoked. In a case in which the patient's name is not in the list, the acupuncturist should accept the patient by tapping "Search Patient" on the menu bar.

### (4) Search Patient (Reception Region)

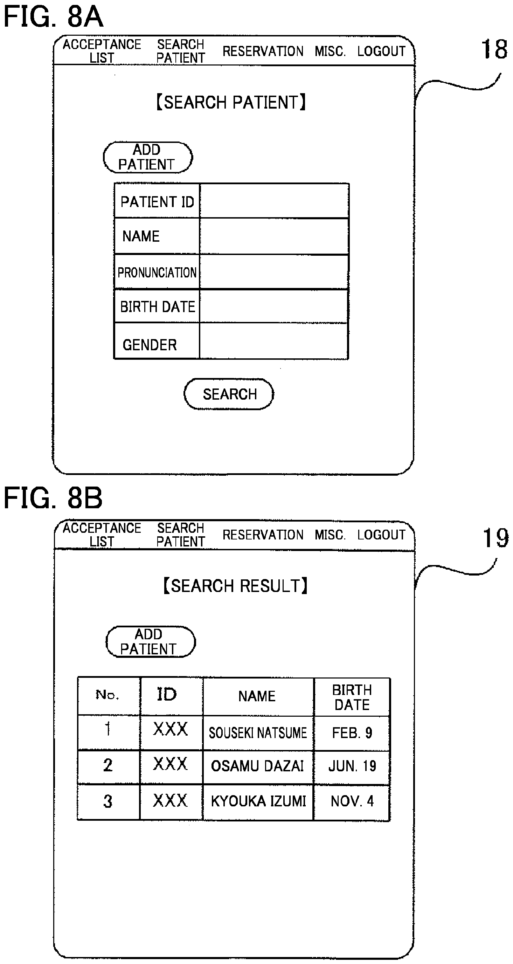

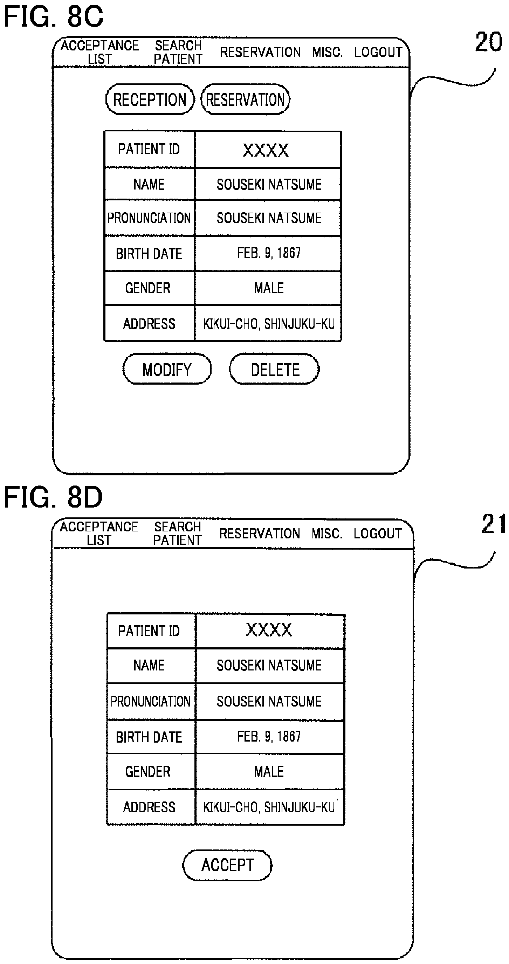

The acupuncturist taps "Search Patient" to open a screen 18 (Fig. 8A), where it is possible to search the patient by the ID or the name thereof. If <Add Patient> is tapped on the screen 18, it becomes possible to register a new patient. In a case in which a search result screen 19 (Fig. 8B) is displayed, upon tapping "Name", a patient master screen 20 (Fig. 8C) is invoked for selection from among <Reception>, <Reservation>, <Modify>, and <Delete>. Upon selecting <Modify> or <Delete> from the patient master screen 20, a user may modify or delete the registered contents displayed in the patient master screen 20. Furthermore, upon tapping <Accept>, a reception confirmation screen 21 (Fig. 8D) is invoked and the reception operation ends.

### (5) Reservation (Reservation Region)

In the following, a description will be given of a procedure for the acupuncturist to register a reservation for a patient in two cases consisting of an already accepted patient and a new patient.

### (a) Case of Already Accepted Patient

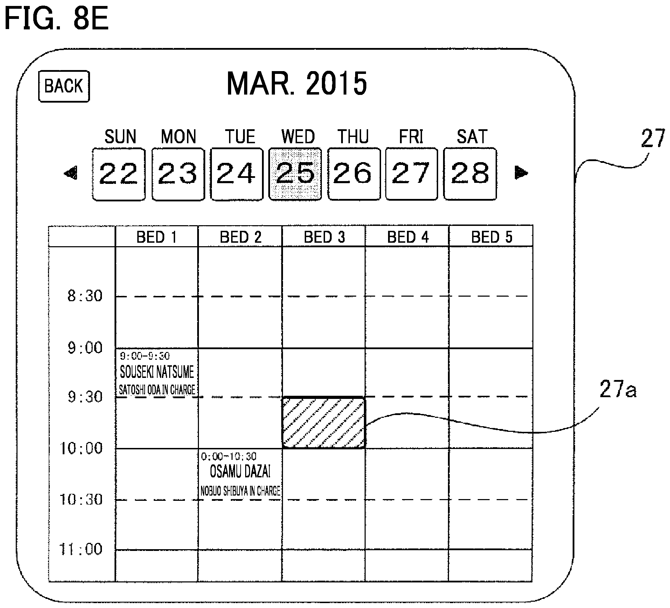

The acupuncturist taps the above described "Acceptance List" to open the list of accepted patients of the screen 17, taps a specific patient from among the patient list to open the patient master screen 20 (Fig. 8C), taps "Reservation" of the screen 20 to open the reservation calendar 27 (Fig. 8E), and inputs information on a reservation for treatment in a field 27a of a time available and desired by the patient in the reservation calendar 27. Upon tapping "Back" to return to the patient master screen 20, registration of the reservation is complete. As already described, the patient master screen 20 may be opened from the search result screen 19.

### (b) Case of New Patient

In a case of a new patient, the acupuncturist taps "Search Patient" to open the screen 18, and, before inputting a name and the like of the new patient, taps "Reservation" to open the reservation calendar 27. The acupuncturist inputs information on a reservation for treatment in the field 27a of a time available and desired by the new patient, then, taps "Back" to return to the screen 18, inputs name and the like of the new patient, and finally taps "Add Patient". In this case, upon tapping "Add Patient", the name and the like of the new patient are at the same time inputted in the field 27a where the reservation has been registered. Thus, the registration of the new patient and the registration of the reservation of the new patient are simultaneously complete.

### (6) Interview Sheet Input (Questionnaire Region, Subjective Assessment Region)

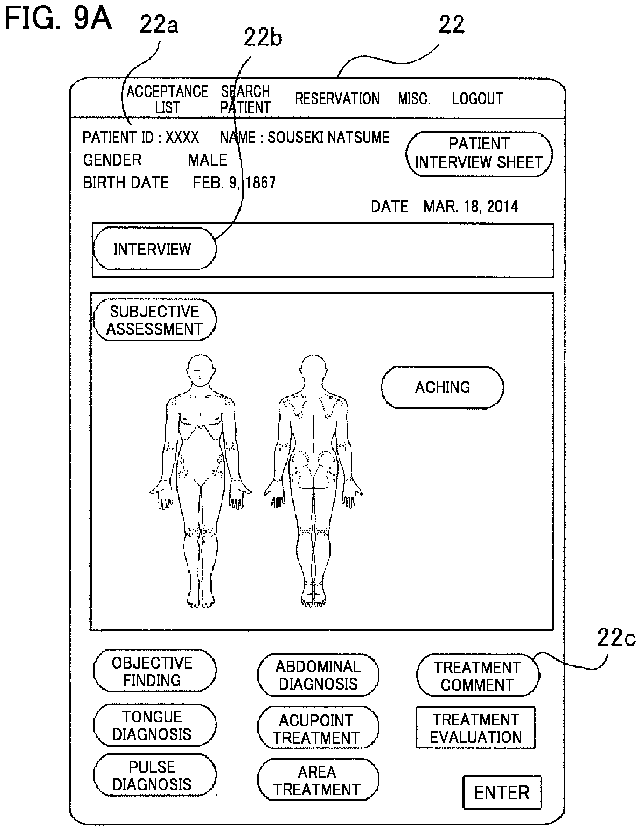

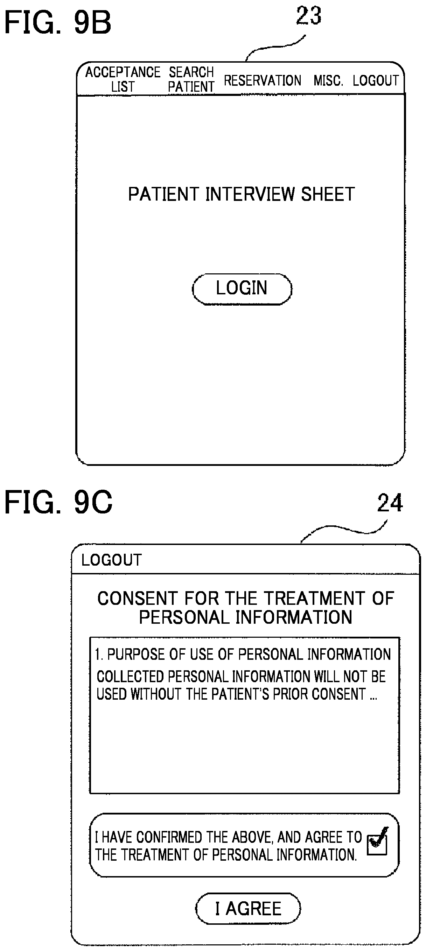

To prepare for a treatment, the acupuncturist taps "Name" of the patient to be treated in the list of the accepted patients (Fig. 7C). Since a patient medical chart 22 (Fig. 9A) for the patient is opened from among the electronic medical charts 50, the acupuncturist taps <Patient Interview Sheet> in the patient medical chart 22 to log-in from a screen 23 (Fig. 9B) to the patient region 53. Then, the acupuncturist hands the tablet terminal 100 to the patient and lets the patient input items of initial visit questionnaire and subjective assessment.

### (7) Patient Medical Chart

The treatment starts when the patient enters in the consulting room. In the patient medical chart 22 (Fig. 9A), the following items are displayed sequentially downward.
(a) Patient Information 22a (ID, Name, Gender, Birth Date)
(b) Questionnaire Button 22b (for invoking the contents of the patient's input in the patient region)
(c) Buttons 22c for selection from among the subjective assessment region 53b, the objective finding region 51a, the tongue diagnosis region 51b, the pulse diagnosis region 51c, the abdominal diagnosis region 51d, the acupoint treatment region 51e, the area treatment region 51f, the treatment comment region 51g, and the treatment evaluation region 53c.

Although transition from the acupuncturist region 51 to the patient region 53 is automatic, entry of a four digit PIN (Personal Identification Number) is required to return from the patient region 53 back to the acupuncturist region 51, for the purpose of access restriction against the patient, who has finished filling the interview sheet.

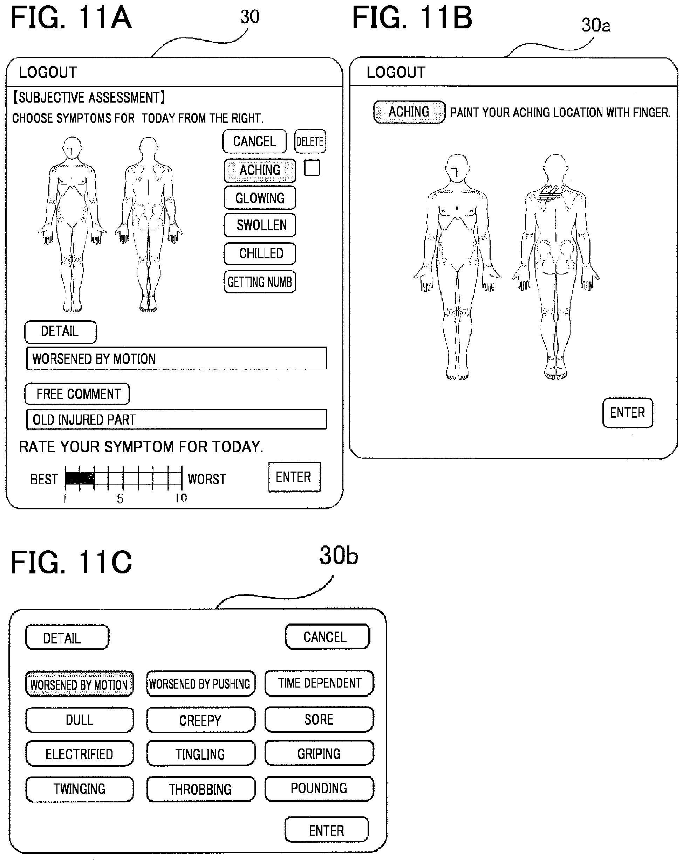

The acupuncturist logs in to the patient region 53, and, when a screen 24 (Fig. 9C) of "Consent for the Treatment of Personal Information" is displayed (in a case of a new patient) or a subjective assessment screen 30 (Fig. 11A) is displayed (in a case of a patient for reexamination), the acupuncturist hands the tablet terminal 100 to the patient to continue the input.

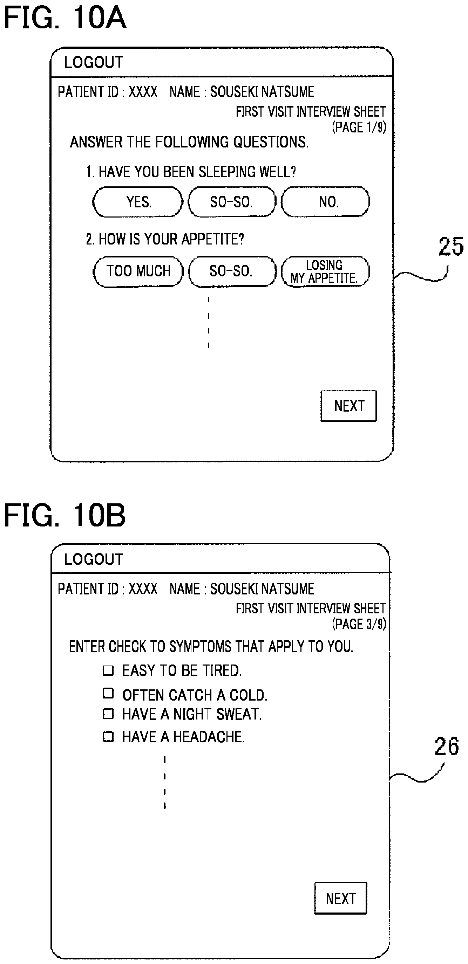

The initial visit questionnaire includes two types of screens: a button selection type screen 25 (Fig. 10A); and a check box selection type screen 26 (Fig. 10B), both of which allow the patient to input each item by tapping alone. The patient region 53 is displayed in a "patient-oriented" form dedicated to easy input. Also the acupuncturist region 51 allows a user to display and input the contents of the patient's input in the questionnaire. When the new patient finishes filling the initial visit questionnaire, the screen goes to the subjective assessment screen 30 (Fig. 11A). The subjective assessment region 53b is configured by the medical chart module.

### <Description of Regions of Electronic Medical Charts of Acupuncture and Moxibustion Treatment Support Terminal Device>

In the following, descriptions will be given of the regions of the electronic medical charts 50 of the acupuncture and moxibustion treatment support terminal device according to the present embodiment.

### <Subjective Assessment Region>

The patient selects and taps a symptom from among a list of symptom buttons in the subjective assessment screen 30 (Fig. 11A). For example, if <Aching> is tapped, a full body schema screen 30a (Fig. 11B) is widely displayed, and the patient can show the area that aches by painting. When the patient finishes painting and taps <Enter>, the screen goes back to the subjective assessment screen 30 (Fig. 11A), showing a schema similarly painted, thereby allowing the user to confirm the aching location. On the right of the <Aching> button, a check box is displayed to indicate that the <Aching> button has been already selected and inputted. Upon entering a check to the check box and tapping <Delete>, the input of <Aching> is deleted.

If a <Detail> button at a center of the subjective assessment screen 30 (Fig. 11A) is tapped, a button tool screen 30b (Fig. 11C) of the medical chart module is invoked. For example, upon tapping <Worsened by motion> and then <Enter>, a text "Worsened by motion" is inputted in a text input field below the <Detail> button of Fig. 11A.

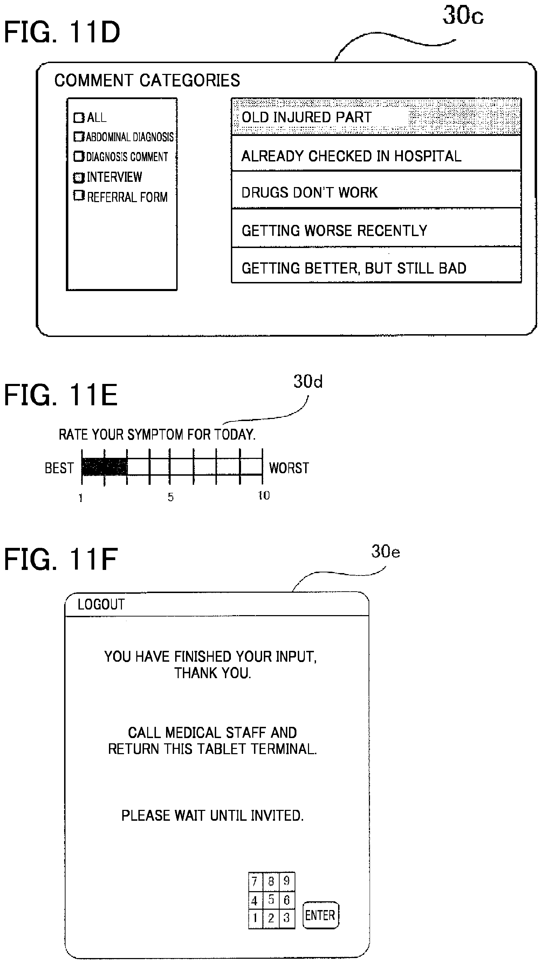

If a <Free Comment> button in the subjective assessment screen 30 (Fig. 11A) is tapped, the comment module is activated to display a screen 30c (Fig. 11D). The patient can select and input one of the predefined texts that have been prepared in advance. For example, if the patient taps a predefined text "Old injured part" registered in advance in the screen 30c (Fig. 11D), the text "Old injured part" is inputted in a text input field below the <Free Comment> button in the subjective assessment screen 30 (Fig. 11A). The patient can freely append to and edit the texts in the text input fields described above from the keyboard.

A slider 30d (Fig. 11E) at a lower part of the subjective assessment screen 30 (Fig. 11A) allows the patient to input a visual analog scale (Hereinafter, referred to as a "VAS"). The patient inputs an extent of the symptom by sliding a finger on the slider 30d. Since the patient is supposed to input the VAS at every visit, the VAS is a useful indicator of chronological change in treatment evaluation. Also, at the time of the treatment evaluation after the treatment, the patient is supposed to input again the VAS by means of the slider tool in the same manner. Accordingly, it is possible for the acupuncturist and the patient to compare a change in VAS and confirm a short term effect of the treatment.

When the patient finishes filling the entire interview sheets (the questionnaire region, the subjective assessment region), a screen 30e (Fig. 11F) is displayed to request the patient to return the tablet terminal 100 to the acupuncturist. The acupuncturist, having the tablet terminal 100 returned from the patient, inputs the four digit PIN to go back to the patient medical chart 22 (Fig 9A) of the acupuncturist region 51.

### <Objective Finding Region>

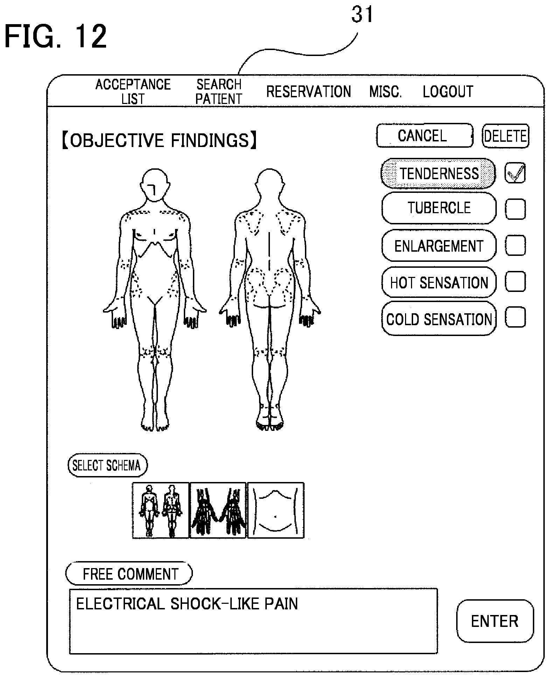

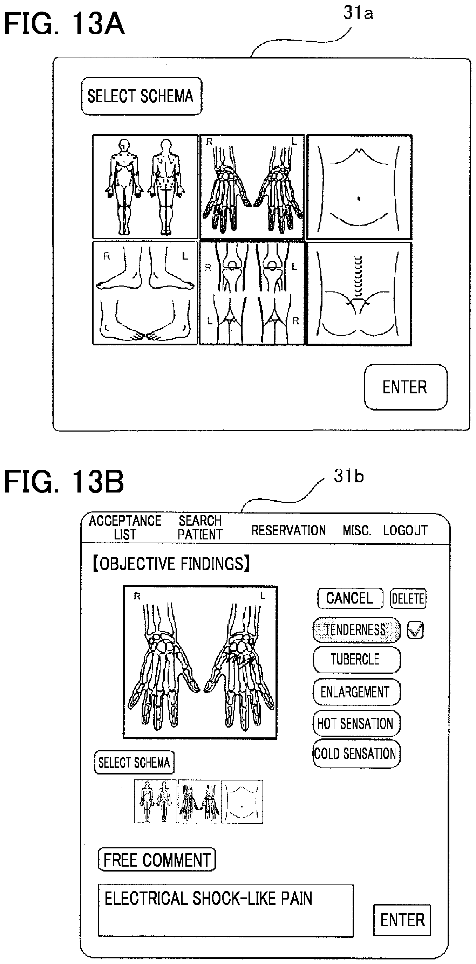

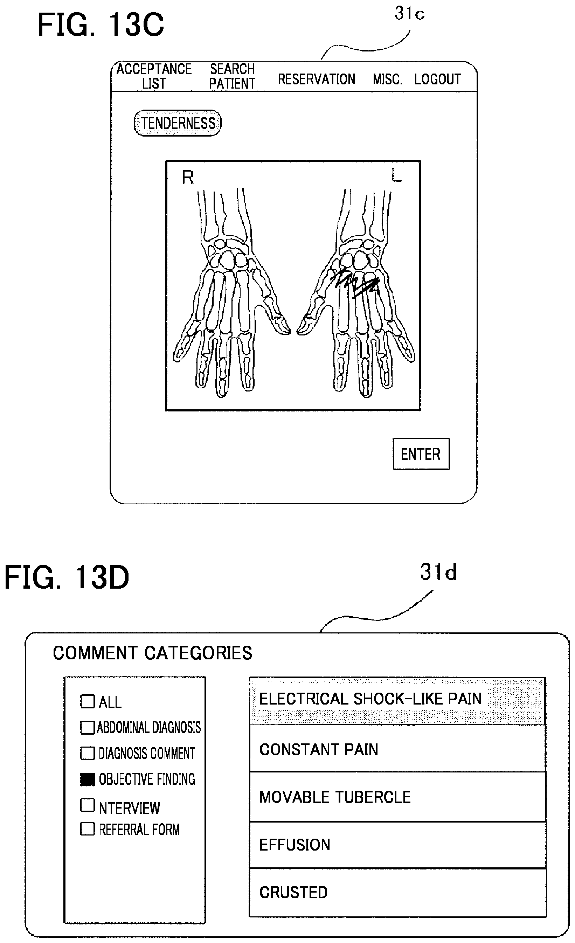

Firstly, the acupuncturist operates the tablet terminal 100 to keep a record in the objective finding region 51a. This means that the acupuncturist taps <Objective Findings> in the patient medical chart 22 (Fig. 9A) to invoke a screen 31 (Fig. 12) of the objective finding region 51a. The objective finding region 51a is configured by the medical chart module. The acupuncturist selectively switches a plurality of schemata by means of the button tool, inputs a category of an objective finding and an area in which the finding has been made by means of the paint tool, and inputs a free comment by means of the text tool. To start with this, the acupuncturist selects the schema. The acupuncturist taps <Select Schema> in a middle part of the screen 31 to display a schema selection screen 31a (Fig. 13A), selects a desired schema and taps <Enter> to switch to a screen 31b (Fig. 13B) in which the selected schema is displayed in close-up. Next, the acupuncturist selects the category of the objective finding. The acupuncturist taps one of objective finding buttons arrayed on a right side of the screen 31b (Fig. 13B) to move to a paint input screen 31c (Fig. 13C). The acupuncturist paints with a finger on the paint input screen 31c, thereby inputting the area of the objective finding, and taps <Enter> to return to the screen 31b (Fig. 13B). In this manner, the area of the finding that has been just inputted by painting is shown in the schema.

Then, the acupuncturist taps <Free Comment> on the screen 31b (Fig. 13B) to invoke a comment module screen 31d (Fig. 13D). The acupuncturist taps one of the predefined texts that have been inputted in advance to insert the predefined text in a text input field in a lower part of the screen 31b (Fig. 13B). The acupuncturist, upon completion of inputting the entire objective findings, taps <Enter> at a bottom right of the screen 31b to finish the objective finding region 51a.

### <Tongue Diagnosis Region>

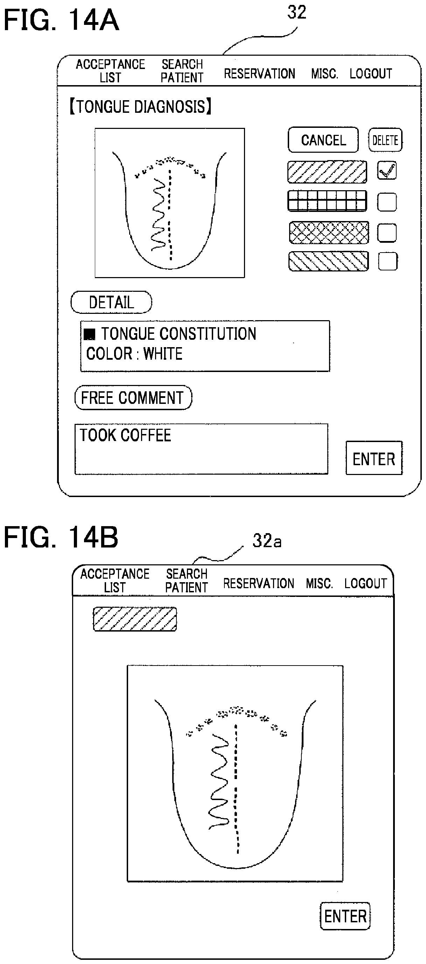

Secondly, the acupuncturist operates the tablet terminal 100 to keep a record in the tongue diagnosis region 51b. To start with this, the acupuncturist taps <Tongue Diagnosis> in the patient medical chart 22 (Fig. 9A) to invoke a tongue diagnosis region screen 32 (Fig. 14A).

The tongue diagnosis region 51b is configured by the medical chart module. The acupuncturist draws an illustration of a tongue configuration by means of the paint tool, selects and inputs a color and a shape of tongue constitution and a color and a condition of tongue coating by means of the button tools, and inputs a free comment by means of the text tool.

The acupuncturist can draw an illustration of the finding on a schema of a tongue by means of the paint tool. The acupuncturist selects and taps a paint color from among buttons arrayed on a right side of the tongue diagnosis region screen 32 (Fig. 14A) to move to a paint input screen 32a (Fig. 14B). The acupuncturist draws with a finger the illustration of the finding and taps <Enter> to return to the tongue diagnosis region screen 32 (Fig. 14A). In this manner, the illustration of the finding that has been just inputted is shown in a schema of the screen 32.

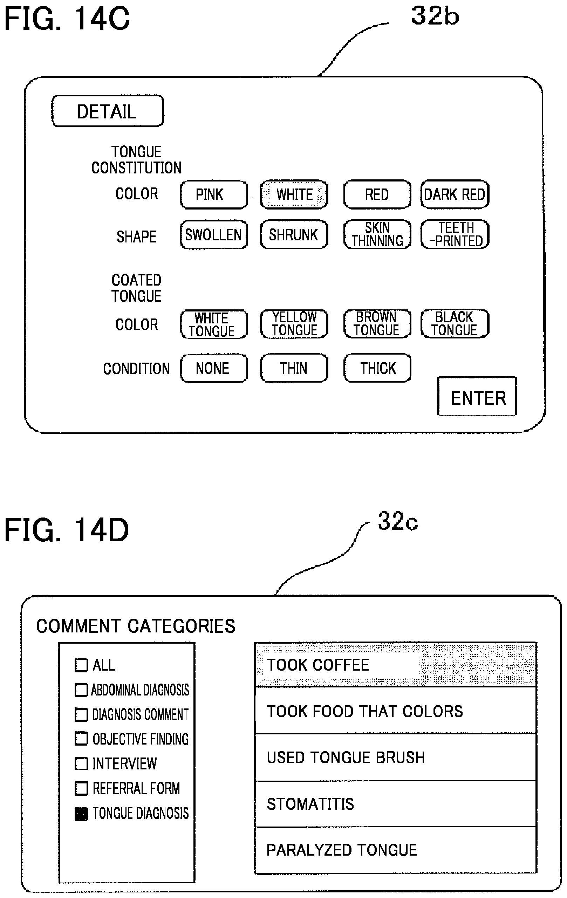

Usually, the tongue diagnosis is expressed in terms of a color and a shape of tongue constitution and a color and a condition of tongue coating, all of which can be selected and inputted by the acupuncturist by means of the button tool. Furthermore, when the acupuncturist taps <Detail> on the screen 32 (Fig. 14A) to invoke a button tool screen 32b (Fig. 14C), the acupuncturist can select a finding button of the tongue diagnosis and tap <Enter> to input the finding as a text in a text input field below the <Detail> button in the screen 32 (Fig. 14A).

It is possible to input other findings and comments by means of the text tool. When the acupuncturist taps <Free Comment> on the screen 32 (Fig. 14A) to invoke a comment module screen 32c (Fig. 14D), the acupuncturist can select and tap one of the predefined texts that have been prepared in advance to insert the predefined text in a text input field on a lower part of the tongue diagnosis screen 32. It is also possible to freely append to and edit the inserted text. The acupuncturist, upon completion of inputting the entire tongue findings, taps <Enter> at a bottom right of the tongue diagnosis region screen 32 to finish inputting items of the tongue diagnosis region 51b.

### <Pulse Diagnosis Region>

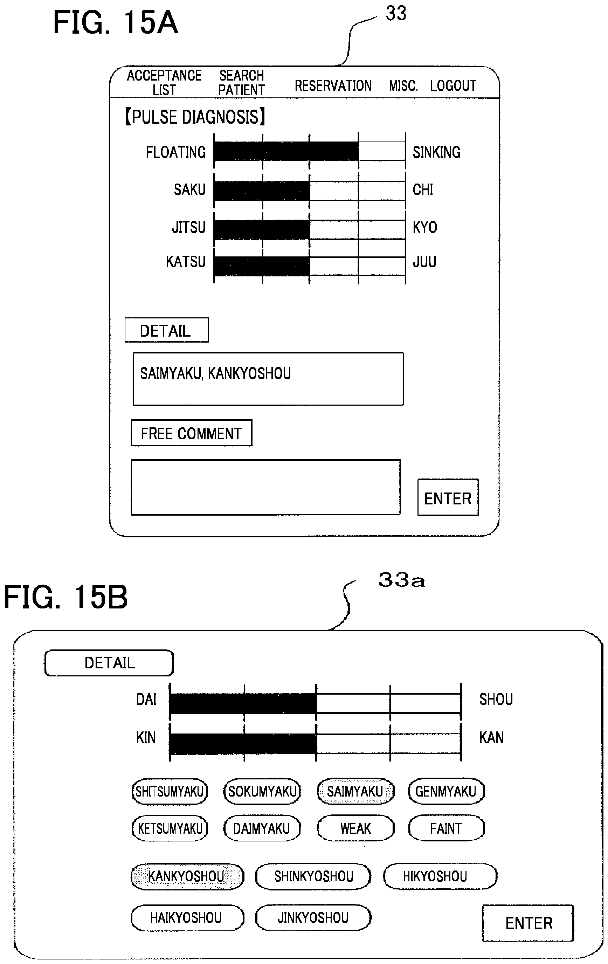

Thirdly, the acupuncturist operates the tablet terminal 100 to keep a record of pulse diagnosis in the pulse diagnosis region 51c. To start with this, the acupuncturist taps <Pulse Diagnosis> in the patient medical chart 22 (Fig. 9A) to invoke a pulse diagnosis region screen 33 (Fig. 15A). The pulse diagnosis region 51c is configured by the medical chart module. The acupuncturist inputs somyaku by means of the slider tool, selects and inputs a pulse condition by means of the button tool, and inputs a free comment by means of the text tool.

As a practice of the pulse diagnosis, findings of the pulse diagnosis are classified into six items of "fuchin", "sakuchi", "kyojitsu", "katsujuu", "daishou", and "kinkan" each having five levels (for example, fuchin has five levels of "floating", "slightly floating", "middle", "slightly sinking", and "sinking"), as well as specifically named pulse conditions such as "genmyaku", "sokumyaku", and "daimyaku". From among the six items, "fuchin", "sakuchi", "kyojitsu", and "katsujuu" are called "somyaku".

At an upper part of the pulse diagnosis region screen 33, four sliders are provided for inputting the four types of "somyaku". The acupuncturist slides each slider with a finger to input one of the five levels. The resultant input via the slider is converted into a text and displayed in a text input field. For example, by sliding the slider slightly toward "floating" from the central position and tapping <Enter>, a text "slightly floating" is inputted in the text input field. In order to input a further detailed pulse condition, the acupuncturist taps <Detail> to display a button tool screen 33a (Fig. 15B). In a case of the pulse diagnosis region, sliders for inputting "daishou" and "kinkan" in five levels are also displayed above the buttons. The acupuncturist selects one of the buttons to input detailed findings of the pulse diagnosis and taps <Enter> to input the findings as a text in a text input field below the <Detail> button of the screen 33.

It is possible to input other findings, comments, and the like by means of the text tool. For example, the acupuncturist taps <Free Comment> on the screen 33 to invoke a comment module screen. The acupuncturist selects and taps one of the predefined texts that have been prepared in advance to insert the predefined text in a text input field on a lower part of the screen 33 of the pulse diagnosis region 51c. It is possible to freely append to and edit the inserted text. The acupuncturist, upon completion of inputting the entire pulse findings, taps <Enter> at a bottom right of the pulse diagnosis region screen 33 to finish the pulse diagnosis region 51c. Each piece of numeric information inputted by means of the slider tool is converted into text information such as, for example, "slightly floating", and recorded in the patient medical chart 22.

### <Abdominal Diagnosis Region>

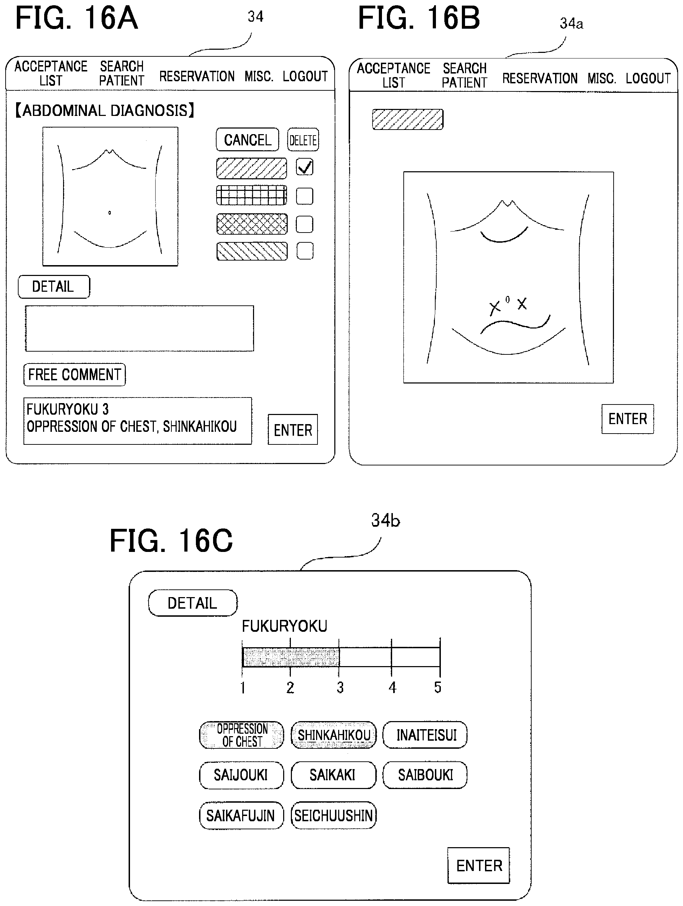

Fourthly, the acupuncturist operates the tablet terminal 100 to keep a record in the abdominal diagnosis region 51d. To start with this, the acupuncturist taps <Abdominal Diagnosis> in the patient medical chart 22 to display an abdominal diagnosis region screen 34 (Fig. 16A). The abdominal diagnosis region 51d is configured by the medical chart module. The acupuncturist draws an illustration of contents and areas of findings by means of the paint tool, selects and inputs the findings of the abdominal diagnosis by means of the button tool, and inputs a free comment by means of the text tool.

It is possible for the acupuncturist to draw an illustration of the findings of the abdominal diagnosis by means of the paint tool. In this case, the acupuncturist selects and taps a paint color from among buttons on a right side of the abdominal diagnosis region screen 34 to move to a paint input screen 34a (Fig. 16B). The acupuncturist draws with a finger the illustration of the findings and taps <Enter> to return to the abdominal diagnosis region screen 34. In this manner, the illustration of the finding that has been just inputted is shown in a schema of the screen.

Furthermore, it is possible for the acupuncturist to input detailed findings of the abdominal diagnosis by means of the button tool. In this case, the acupuncturist taps <Detail> on the screen 34 to display a button tool screen 34b (Fig. 16C). In a case of the abdominal diagnosis region 51d, a slider for inputting "fukuryoku" in five levels is also displayed above the buttons. The acupuncturist selects one of the buttons and taps <Enter> to input the finding as a text in a text input field below the <Detail> button of the screen 34.

It is possible to input other findings and comments and the like by means of the text tool. In this case, the acupuncturist taps <Free Comment> on the screen 34 to invoke a comment module screen. The acupuncturist selects and taps one of the predefined texts that have been prepared in advance to insert the predefined text in a text input field on a lower part of the screen 34 of the abdominal diagnosis region 51d. It is possible to freely append to and edit the inserted text. The acupuncturist, upon completion of inputting the entire abdominal findings, taps <Enter> at a bottom right of the abdominal diagnosis region screen 34 to finish the input of the abdominal findings.

In the above, oriental medical findings including the tongue diagnosis, the abdominal diagnosis, the pulse diagnosis, and the like have been described. However, it should be noted that the oriental medical findings may include characteristic bodily findings of traditional medicines of nations such as Japan, China, Korea, India, and the like.

### <Acupoint Treatment Region>

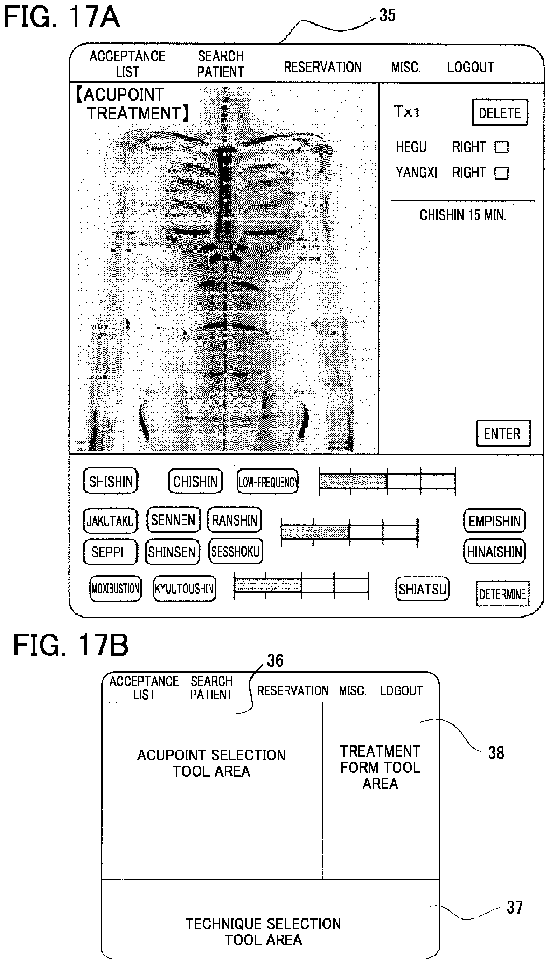

Acupuncture treatment includes treatment of a "point" such as an acupoint and treatment of a "surface" such as pediatric acupuncture treatment. In the acupoint treatment region 51e, the acupuncturist can easily record treatment against any of 361 acupoints defined by World Health Organization (herein referred to as "WHO"). The acupuncturist taps <Acupoint Treatment> on the patient medical chart 22 to invoke a screen 35 (Fig. 17A) of the acupoint treatment region 51e.

In a usual treatment, the acupuncturist performs a plurality of techniques in sequence. Hereinafter, each single technique is referred to as a unit of treatment Txn ("n" denotes an integer). For example, the techniques are classified and recorded in a manner such that "chishin" for Tx1, "kyuutoushin" for Tx2, and "hinaishin" for Tx3.

The screen of the acupoint treatment region 51e is configured by an acupoint selection tool, a technique selection tool, and a treatment form tool, which are respectively assigned on the screen to an acupoint selection tool area 36, a technique selection tool area 37, and a treatment form tool area 38 (Fig. 17B). Each unit of treatment Txn is recorded in the following manner.
(a) The acupuncturist selects the acupoints used in the treatment by means of the acupoint selection tool.
(b) The acupuncturist selects the technique and inputs amounts (duration, intensity, number of moxibustion) of the treatment by means of the technique selection tool.
(c) The acupuncturist performs a minor modification for each unit of treatment such that, for example, the acupoints on which side (right, left, or both) have been used, whether the treatment has been supplied or reduced, and/or the like, and issues a record of each unit of treatment by means of the treatment form tool.

In the following, a detailed description will be given of the acupoint selection tool, the technique selection tool, and the treatment form tool.

### (1) Acupoint Selection Tool

In the acupoint selection tool area 36, the acupuncturist selects at least one acupoint for each unit of treatment Txn by means of the acupoint selection tool. The acupuncturist is provided with three selection methods of; (a) selecting and tapping an acupoint mapped on a virtual 3D (three dimensional) human body model of meridians and acupoints; (b) selecting and tapping an acupoint name on a list of meridians and acupoints; and (c) selecting and tapping an acupoint on a two dimensional illustration of meridians and acupoints. The three selection methods will be described below.

### (a) Method by Using Virtual 3D Human Body Model

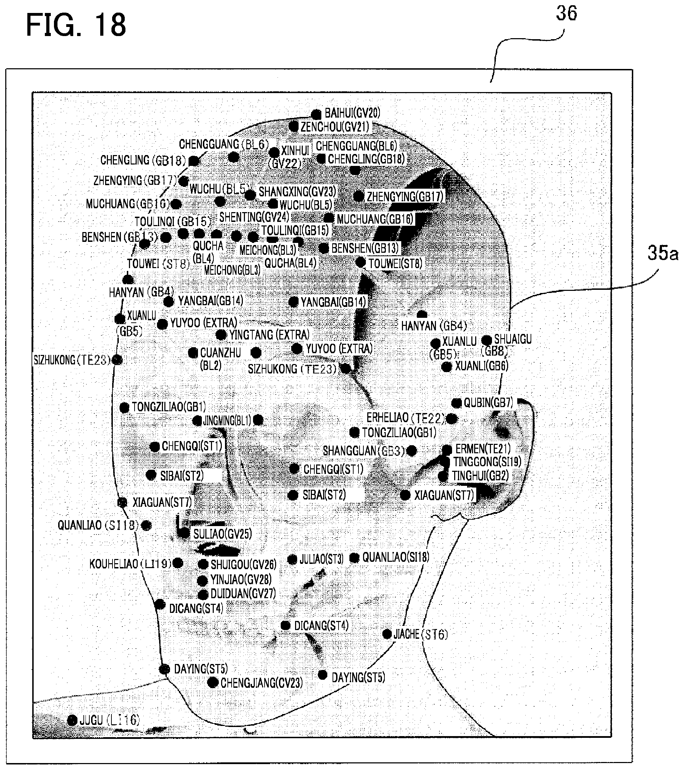

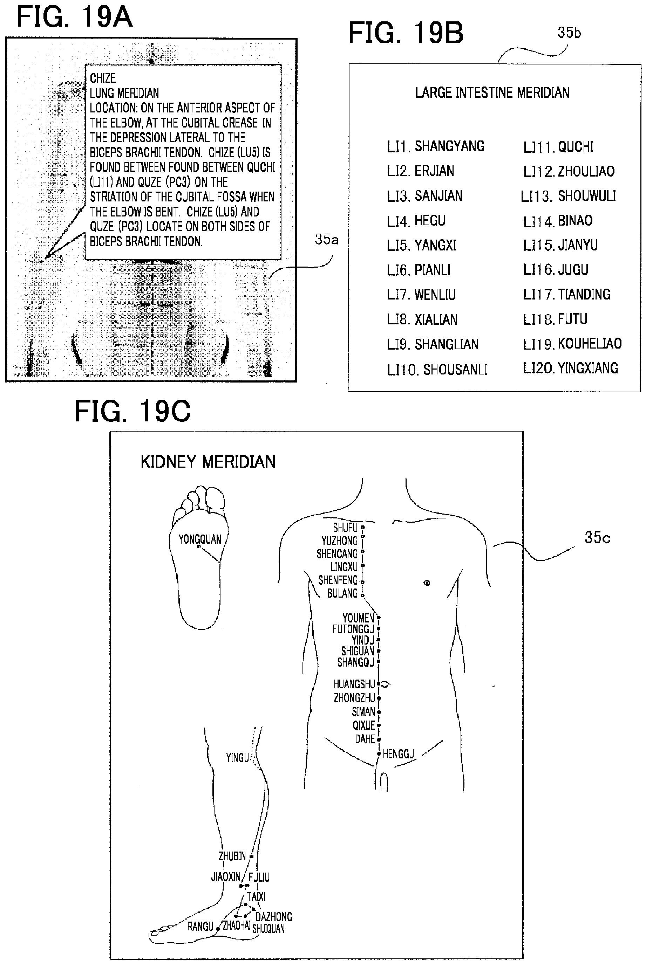

This method makes it possible for the acupuncturist to select an acupoint mapped on a virtual 3D polygon of the human body model. Since the 3D human body model 35a (Fig. 18) is created anatomically accurate and connective tissues such as muscles and tendons, vascular channels, and bones are transparently visible, the acupuncturist can easily locate an acupoint in three dimensions. In the 3D human body model 35a, names and codes of the acupoints are displayed as shown in Fig. 18. For example, in a part of the jaw, a name "Daying" and a code "ST5" of an acupoint are displayed on each of right and left sides. Since it is possible to shrink, enlarge, rotate, and drag with a finger on the 3D human body model 35a, the acupuncturist can easily approach a desired acupoint. Furthermore, by configuring in advance to display information related to the selected acupoint, the acupuncturist can confirm the information (such as a meridian which the acupoint belongs to, an alias, a method of finding the acupoint, and effects) related to the acupoint while selecting the acupoint at a time of selection thereof. Fig. 19A shows a state of displaying the information related to the acupoint selected on the 3D human body model 35a. Information such as the 3D human body model 35a, the names and the codes of the acupoints, and location marks of the acupoints may be stored in a storing unit included in the tablet terminal 100 or may be supplied from another terminal or an outside server.

### (b) Method by Using Meridians and Acupoints List

In the acupoint selection tool area 36, a list 35b (Fig. 19B) of meridians and acupoints is arrayed, and the acupuncturist taps and selects an acupoint name on the list 35b. In the list 35b, the acupoints are classified for each meridian. This method makes it possible for a skilled acupuncturist to select an acupoint faster than selecting the acupoint from the 3D human body model 35a.

### (c) Method by Using Illustrations of Meridians and Acupoints

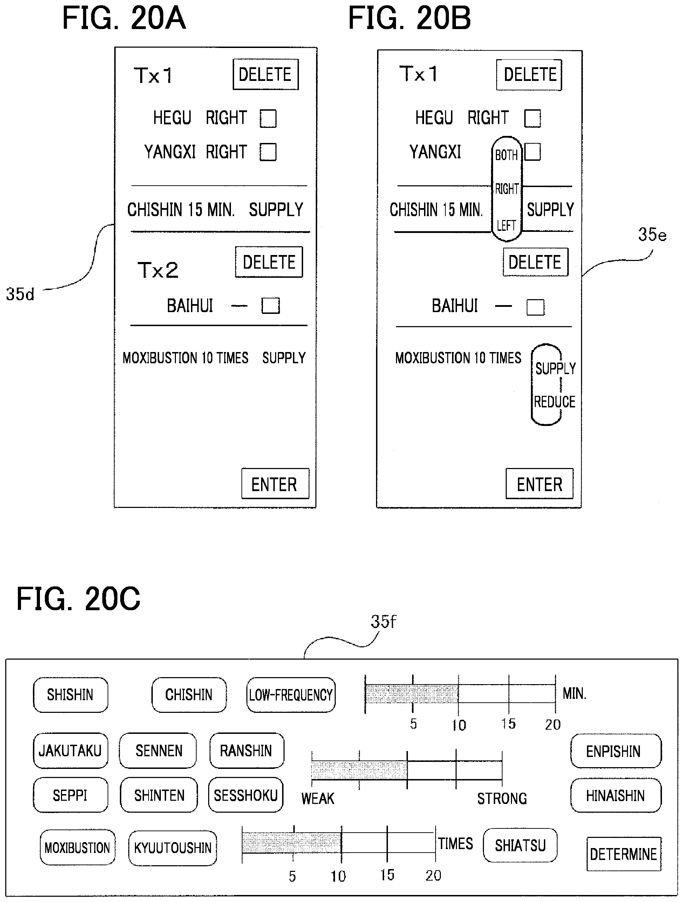

The acupuncturist selects an acupoint from an illustration 35c (Fig. 19C) showing acupoints on a schematic drawing of the human body. The illustration 35c can be selectively shrunk and/or enlarged by closing and/or opening two fingers ("pinch-in" and/or "pinch-out"), and can be moved by dragging the illustration 35c. The acupuncturist can select an acupoint by tapping the acupoint on the illustration 35c. Since illustrations similar to the illustration 35c are generally used and well known in regular textbooks on meridians and acupoints, the acupuncturist can easily use the illustration 35c to find acupoints. The acupoints selected by the above described methods are listed in the treatment form tool area 38 (Fig. 20A).

### (2) Technique Selection Tool

The acupuncturist selects a technique for each unit of treatment from the technique selection tool area 37. The techniques can be categorized into a type of techniques specified by a name thereof alone (such as shishin, enpishin, hinaishin, and shiatsu), a type of techniques specified along with duration (such as chishin and low frequency acupuncture therapy), a type of techniques specified along with intensity (such as jakutakujutsu, sennenjutsu, ranshin, seppi, sintenjutsu, and sesshokushin), and a type of techniques specified along with a number of moxibustion (such as moxibustion and kyuutoushin). In a technique selection tool screen 35f (Fig. 20C), the acupuncturist inputs the technique by means of the button tool, and inputs the duration, the intensity, and the number of moxibustion by means of the slider tool. The inputted technique is recorded in the treatment form tool area 38. The acupuncturist taps a <Determine> button on the screen 35f to finish inputting the current unit of treatment Txn. Subsequently, the acupuncturist inputs the next unit of treatment Tx(n+1) by means of the acupoint selection tool.

### (3) Treatment Form Tool

The acupuncturist can perform minor modifications on the inputted units of treatment Txn by means of the technique selection tool (Figs. 20A and 20B). The acupuncturist can delete an erroneously inputted acupoint by entering a check to a check box on a right side of a listed name of the acupoint and tapping a <Delete> button.

There are acupoints having left-right distinction and acupoints located on a midline and therefore having no left-right distinction. An indication indicative of "both sides", "left side", or "right side" is displayed on a right side of a listed name of the acupoint in the screen 35d (Fig. 20A). When the acupuncturist taps the indication, viz., a drop down list of "both sides", "left side", or "right side" used to select any one of them is displayed on a right side of a listed name of the acupoint in the screen 35e (Fig. 20B).

Furthermore, some techniques have supply-reduce distinction. An indication indicative of "Supply" or "Reduce" is displayed on a name of the type of the technique. When the acupuncturist taps the indication, viz., a drop down list of "Supply" and "Reduce" to select any one of them is displayed on a right side of a listed name of the type of the technique in the screen 35e (Fig. 20B).

As described above, the acupuncturist inputs the unit of treatment Txn in sequence such as Tx1, Tx2, and so forth by means of the acupoint selection tool and the technique selection tool, and then performs the minor modification by means of the treatment form tool. Finally, the acupuncturist taps <Enter> (Figs. 20A or 20B) to complete the acupoint treatment region. In this manner, contents of the treatment are recorded in the patient medical chart 22 as text information.

### <Area Treatment Region>

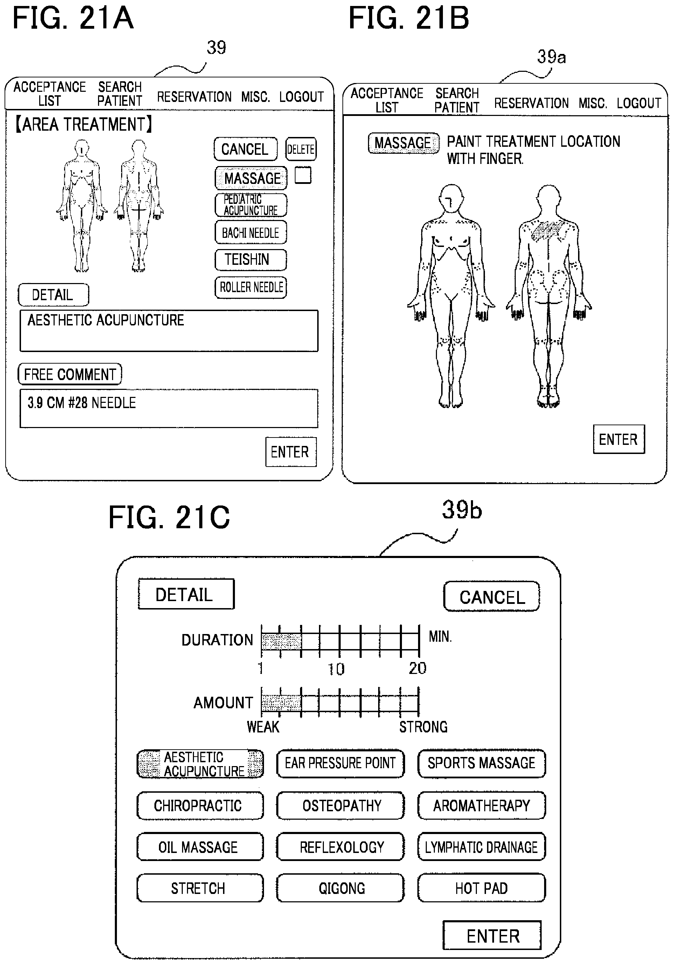

As already described above, the acupuncture treatment includes treatment of a "point" (i.e., an acupoint) and treatment of a "surface" such as pediatric acupuncture. In the area treatment region 51f, the acupuncturist can record the treatment of the "surface". The acupuncturist taps <Area Treatment> in the patient medical chart 22 to invoke a screen 39 (Fig. 21A) of the area treatment region 51f.

The area treatment region 51f is configured by the medical chart module. The acupuncturist inputs a category and areas of the treatment by means of the paint tool, inputs duration and amounts of the treatment by means of the slider tool, and inputs a detailed content of each technique by means of the button tool.

Firstly, the acupuncturist selects and taps a category of the technique from the list of category buttons on a right side of a screen 39 (Fig. 21A) of the area treatment region 51f. For example, in a case in which the acupuncturist taps a <Massage> button, a screen 39a (Fig. 21B) is invoked in which a full body schema is widely displayed, where the acupuncturist can paint the massaged area. The acupuncturist, upon completion of the painting, taps <Enter> to return to the screen 39 (Fig. 21A), where a schema is displayed similarly painted so that the acupuncturist can confirm the massaged area.

In this case, a checkbox is displayed on a right side of the <Massage> button to indicate that the button has been selected and inputted. If a check is entered to the checkbox and <Delete> is tapped, the input for <Massage> is deleted.

If the acupuncturist taps a <Detail> button at a center of the screen 39, a slider tool and button tool screen 39b (Fig. 21C) of the medical chart module are invoked. It is possible to input a duration and amounts of the treatment by means of the slider tool, and to input a detailed content of the treatment by means of the button tool. For example, by tapping <Aesthetic Acupuncture> and then <Enter>, a text "Aesthetic acupuncture" is inputted in a text input field below the <Detail> button of the screen 39 (Fig. 21A).

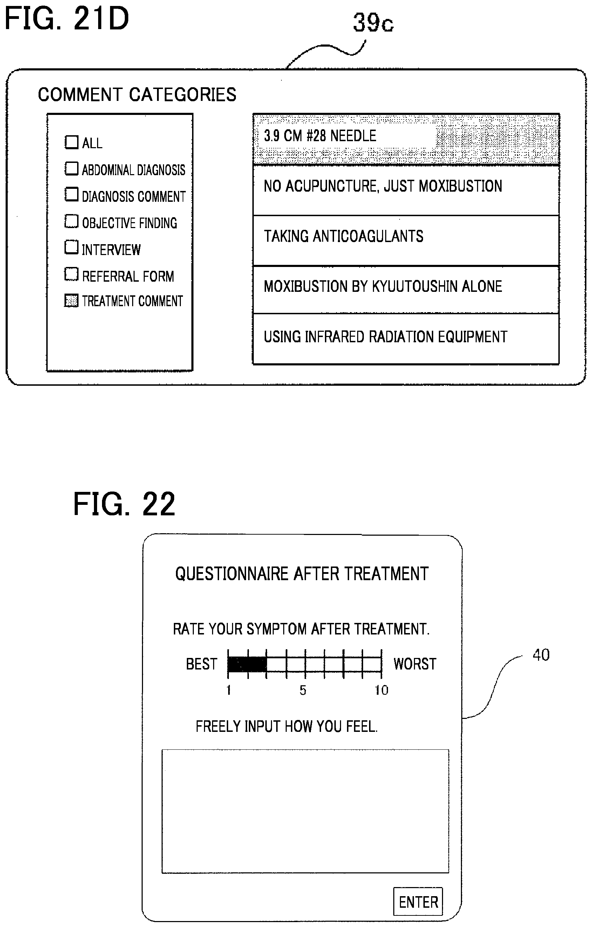

Secondly, if the acupuncturist taps a <Free Comment> button, a comment module screen 39c is invoked (Fig. 21D). When the acupuncturist selects and taps one of the predefined texts that have been inputted in advance, the selected predefined text is inserted in a text input field at a lower part of the screen 39 of the area treatment region. In this manner, the acupuncturist can freely append to and edit the inserted text.

The acupuncturist, upon completion of inputting the techniques for entire treated areas, taps <Enter> at a bottom right of the screen 39 of the area treatment region 51f to finish the area treatment region 51f.

### <Treatment Comment Region>

The acupuncturist taps a <Treatment Comment> button on the patient medical chart 22 (Fig.9A) to invoke a screen (not shown) of the treatment comment region 51g. In the treatment comment region 51g, the acupuncturist can input with a keyboard general remarks on the treatment and contents of the treatment unfit for inputting in the acupoint treatment region 51e or the area treatment region 51f. The acupuncturist can activate the comment module and tap and input the frequently used phrases and sentences that have been registered in advance.

### <Treatment Evaluation Region>

The acupuncturist taps <Treatment Evaluation> in the patient medical chart 22 to login to a screen 40 (Fig. 22) of the treatment evaluation region 53c, which belongs to the patient region 53 for the patient to evaluate the acupuncturist's treatment.

The acupuncturist hands to the patient the tablet terminal 100 in a state of being logged in to the treatment evaluation region 53c. The evaluation is performed by the VAS input and a free text input (Fig. 22). Even after the entire input is complete, the patient cannot exit the treatment evaluation region 53c. When an announce screen 30e (Fig. 11F) is displayed, the patient returns the tablet terminal 100 to the acupuncturist, and the acupuncturist inputs the four digit PIN (Personal Identification Number) to return to the acupuncturist region 51. The acupuncturist, upon completion of inputting the entire regions, taps <Enter> at a bottom of the patient medical chart 22 to close the patient medical chart 22.

### <Second Embodiment>

The present embodiment is directed to an exemplary case such as supporting the acupuncturist's treatment using the electronic medical charts 50 of the acupuncture and moxibustion treatment support terminal device.
(1) The acupuncturist as a practitioner is sometimes in need of confirming a character and an explanation of an acupoint during the treatment. Conventionally, the acupuncturist keeps a book or the like close at hand for the reference purpose. However, the acupuncture and moxibustion treatment support terminal device makes it possible for the acupuncturist to display information on an acupoint by tapping the acupoint in the acupoint treatment region 51e of the electronic medical charts 50. Helpful information on acupoints can be easily displayed such as WHO/WPRO (WHO Regional Office for the Western Pacific) compliant codes, names, pronunciation, meridians, locating methods, anatomy (in terms of muscles, nerves, and blood vessels), characters, effects, and combination examples. Thus, the acupuncturist can acquire desired information within a short period of time with precision.
(2) Furthermore, the acupuncturist can retrieve frequently used acupoints for a symptom to be treated using the symptom and an effect related to the treatment as keywords. Not only acupoints as listed in regular books, but also frequently used acupoints updated in real-time can be displayed in ranking order of, for example, use frequency according to clinical data that has been stored in the present system. As a result of this, it becomes possible to improve quality of treatment performed by the acupuncturist.
(3) The acupuncturist, in need of consultation with a doctor, can communicate with the doctor by way of a bulletin board, chatting, and a video phone by means of the tablet terminal 100. Also, the acupuncturist can accurately transmit to the doctor images of a skin condition, how the patient moves, and the like captured by the camera 101. Thus, the acupuncturist can perform more accurate treatment on the patient by collaborating with the doctor.
(4) It is possible for a visually impaired patient to input the patient medical chart to the tablet terminal 100 only by the aid of the tablet terminal 100, especially by the aid of a voice recognition function and a reading function provided to the tablet terminal 100. The tablet terminal 100 can surely support the visually impaired patient incapable of filling subjective symptoms in a paper form.
(5) Using a bulletin board dedicated to acupuncturists as practitioners via the tablet terminal 100, it is possible for the acupuncturists to discuss on various topics of treatment contents, acupuncture society, and the like, which enables collaboration among acupuncturists conventionally staying in respective clinics and isolated from one another. As a result of this, the quality of treatment of the acupuncturists can be improved.

### <Third Embodiment>

The present embodiment is directed to an exemplary case of using the electronic medical charts 50 of the tablet terminal 100 for supporting those who are learning the acupuncture and moxibustion. A description will be given below of a particular example.
(1) By using the above described 3D human body model 35a, the meridians and acupoints list 35b, and the illustration 35c of meridians and acupoints, it is possible for students of an acupuncture and moxibustion school and the like to visually learn the meridians and acupoints. It is possible for the students to learn by tapping answers for quizzes on locations, names, and effects of acupoints as if playing a game. Furthermore, it is possible to list up information to prepare for a national examination such as well-known mnemonic aids for rote learning.
(2) For schools such as an acupuncture and moxibustion school, it is possible to create and distribute video contents and the like to the tablet terminal 100 as the above described acupuncture and moxibustion treatment support terminal device, thereby enabling online learning for users of the tablet terminal 100. The school can manage learning achievement and the like of each student. Furthermore, by providing a social networking service of the acupuncture and moxibustion school and the like, it is possible to deliver public relations and the like from the school to the tablet terminals 100 of the students.

### <Fourth Embodiment>

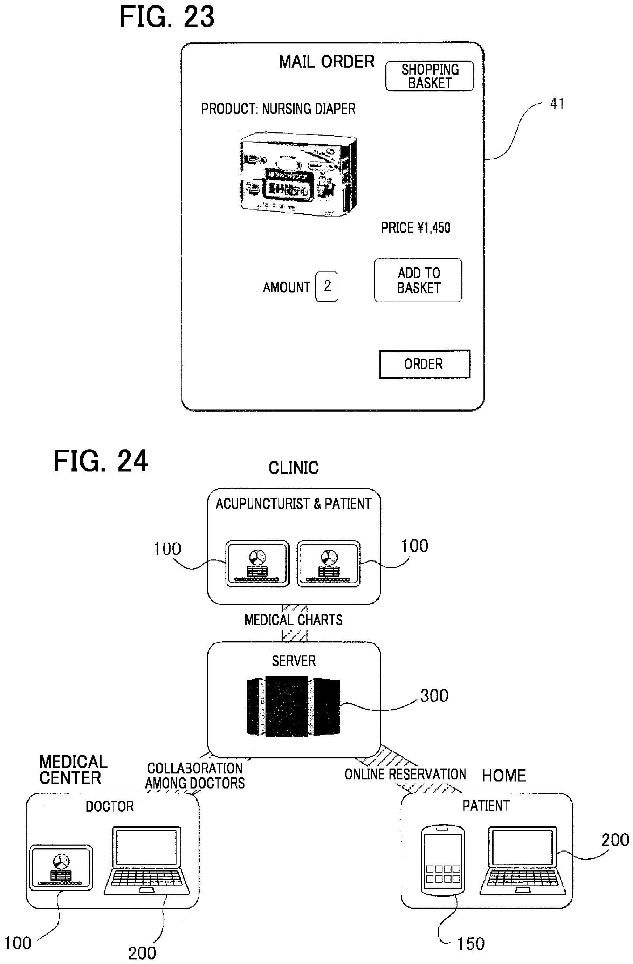

The present embodiment is directed to an exemplary case of adding a mail order function or an advertising medium function to the electronic medical charts 50 of the tablet terminal 100. A description will be given below of a particular example.
(1) The acupuncturist can purchase materials such as a needle and a moxa for the treatment from a catalogue displayed on the tablet terminal 100. Also, the patient can purchase by the mail order various products such as a healthy food and a nursing care product from a screen 41 (Fig. 23) on the tablet terminal 100. The patient can pay for the product at the clinic, and the clinic can have a portion of the profit. To invoke the screen 41 on the tablet terminal 100, the patient, for example, select a button (not shown) to go to a shop displayed on a screen of the tablet terminal 100 such as shown in Fig. 1.
(2) In a waiting room, the patient can read a variety of information. It is possible to earn an advertisement fee by offering a whole or a part of a screen to a particular corporation and/or the like as an advertisement medium displayed for many patients.

### <Fifth Embodiment>

The present embodiment is directed to an example of a medical support system in a case in which the above described tablet terminals 100 respectively provided in a clinic, a patient's home, and a medical support center are connected with one another via the network. Fig. 24 shows an example of the medical support system in which the terminals are connected with one another via the network. As the network, the Internet may be employed. It is possible to realize what is called integrative medicine by the medical support system. Here, the integrative medicine is intended to mean a medicine that coordinates practically and continuously available medical treatments from among the acupuncture, the moxibustion, and any other complementary and alternative medicines in view of values and various backgrounds of the patient. Also, the integrative medicine essentially requires Western medical standby management.

As shown in Fig. 24, in the medical support system according to the present embodiment, a clinic, a medical center, and a home of a patient are connected with one another via a server 300. In the clinic, as described above, the practitioner of the complementary and alternative medicine and the patient creates the patient medical chart 22 constituting the electronic medical charts 50 using the tablet terminal 100. The created patient medical chart 22 is recorded in the server 300. Also, the patient at home can make a treatment reservation on a web site of the clinic by means of a smartphone 150 or a personal computer 200 via the server 300. Furthermore, since the medical center is connected to the server 300, a doctor in the medical center can refer to data of the patient medical chart 22 of the clinic by means of the tablet terminal 100 or a personal computer 200, thereby enabling collaboration between the practitioner in the clinic and the doctor in the medical center, and thus, realizing the integrative medicine. When in need of referral to a local doctor, the doctor in the medical center can write a letter of introduction in his/her name.

As described above, by using the medical support system according to the present embodiment, it is possible to realize the integrative medicine in which the practitioner of the complementary and alternative medicine, the doctor of the medical center, and the patient can collaborate with one another.

### <Sixth Embodiment>

The present embodiment is directed to an exemplary case of using the above described tablet terminal 100 as an aid for the electronic medical charts of a medical institution.

Patient information and the like inputted in the tablet terminal 100 can be outputted to an outside medical institution and integrated into the existing electronic medical charts in the medical institution. In this manner, the aforementioned patient information can become available for the medical institution. For example, it is possible for medical staff members such as a nurse, a physical therapist practicing manual therapy, and a medical technologist and care staff members such as a caregiver to input in the tablet terminal 100 and to output to the electronic medical charts. Furthermore, the medical chart module, with a little modification, can be used for inputting Western medical charts such as a care record and disease activity score of rheumatoid arthritis.

By using the tablet terminal 100 as an aid for the electronic medical charts in the medical institution, it is possible for the medical institution to effectively use the data in the tablet terminal 100, thereby improving the quality of treatment of patients.

### <Seventh Embodiment>

The present embodiment is directed to an exemplary case in which the tablet terminal 100 is used by those who are engaged in other kinds of complementary and alternative medicine.

This means that modification such as adding items related to manual therapy industry such as relaxation service (care) and other kinds of complementary and alternative medicine to the medical chart module can make the tablet terminal 100 applicable in those kinds of industry.

By using the tablet terminal 100 in those kinds of industry, it becomes possible to reliably record and utilize information such as treatment data and data related to those who receive the treatment.

### EXPLANATION OF REFERENCE NUMERALS

- 27: Reservation Calendar
- 50: Electronic Medical Charts
- 51: Acupuncturist Region
- 51a: Objective Finding Region
- 51b: Tongue Diagnosis Region
- 51c: Pulse Diagnosis Region
- 51d: Abdominal Diagnosis Region
- 51e: Acupoint Treatment Region
- 51f: Area Treatment Region
- 51g: Treatment Comment Region
- 52: Clerk Region
- 52a: New Patient Registration Region
- 52b: Reception Region
- 52c: Reservation Region
- 53: Patient Region
- 53a: Questionnaire Region
- 53b: Subjective Assessment Region
- 53c: Treatment Evaluation Region
- 100: Tablet Terminal

## Claims

1. An acupuncture and moxibustion treatment support terminal device, comprising:
a display input unit adapted to display information from and input information to a terminal or a server that stores two-dimensional or three-dimensional information on a human body model in which marks locating human acupoints and names and/or codes of the acupoints are recorded,
wherein the display input unit displays a figure of the human body model on which the marks locating human acupoints and names and/or codes of the acupoints are displayed superimposed at corresponding locations thereof.

2. The acupuncture and moxibustion treatment support terminal device according to Claim 1,
wherein the display input unit displays at least one list of the names and/or the codes of the acupoints in place of the marks locating human acupoints and the names and/or the codes of the acupoints superimposed at the corresponding locations thereof.

3. The acupuncture and moxibustion treatment support terminal device according to Claim 1 or 2,
wherein the display input unit displays an image of an input tool for treatment, the input tool including an input button or an input slider, and, by operating the input button or the input slider, the contents of the treatment are stored and displayed on the display input unit along with the marks locating human acupoints and the names and/or the codes of the acupoints.

4. The acupuncture and moxibustion treatment support terminal device according to Claim 1 or 2,
wherein the acupuncture and moxibustion treatment support terminal device further comprises an image capture unit for capturing an image,
an image acquisition button for causing the image capture unit to start image capturing is displayed, and,
by operating the image acquisition button, the image captured by the image capture unit is stored and displayed on the display input unit along with the marks locating human acupoints and the names and/or the codes of the acupoints.

5. The acupuncture and moxibustion treatment support terminal device according to any one of Claims 1 to 4,
wherein the figure of the two-dimensional or three-dimensional human body model displayed on the display input unit can be enlarged, shrunk, rotated, or moved by operating with a finger a screen of the display input unit.

6. The acupuncture and moxibustion treatment support terminal device according to Claim 1,
wherein the acupuncture and moxibustion treatment support terminal device stores a plurality of figures each showing a part of the human body,
the display input unit displays the figures and a plurality of buttons for selecting symptoms, and, after a predetermined figure is selected from among the figures, an affected area is inputted in and displayed on the display input unit by selecting one of the buttons, and drawing the affected area with a finger on the predetermined figure.

7. An acupuncture and moxibustion treatment support terminal device, comprising:
a display input unit adapted to display information from and input information to a terminal or a server that stores information on a plurality of figures each showing a part of the human body,
wherein the display input unit displays the figures and a plurality of buttons for selecting symptoms, and, after a predetermined figure is selected from among the figures, an affected area is inputted in and displayed on the display input unit by selecting one of the buttons and drawing the affected area with a finger on the predetermined figure.

8. The acupuncture and moxibustion treatment support terminal device according to Claim 6 or 7,
wherein the display input unit displays a field for inputting a comment.

9. The acupuncture and moxibustion treatment support terminal device according to Claim 8,
wherein the acupuncture and moxibustion treatment support terminal device stores a plurality of predefined texts, and a comment selected from among the predefined texts is inputted in the field.

10. The acupuncture and moxibustion treatment support terminal device according to Claim 6 or Claim 7,
wherein the acupuncture and moxibustion treatment support terminal device can display a selection button for selecting and inputting an oriental medical opinion on a patient, a selection button for selecting and inputting information on a color and a shape related to the oriental medical opinion, and a field having a comment on the oriental medical opinion.

11. The acupuncture and moxibustion treatment support terminal device according to Claim 6 or 7,
wherein the display input unit displays a human full body shape and a plurality of buttons for selecting a treatment for a predetermined human body area, and, by selecting one of the buttons to select the treatment and drawing the human full body shape, a location where the selected treatment has been performed is inputted in and displayed on the display input unit.

12. The acupuncture and moxibustion treatment support terminal device according to Claim 11,
wherein the display input unit displays a plurality of buttons for selecting a treatment for another human body area different from the predetermined human body area, sliders for inputting duration and amounts of a treatment, and a field for inputting a comment on the treatment, a treatment for the another human body area and duration and amounts of the treatment are inputted in and displayed on the display input unit by operating the buttons for selecting the treatment for the another human body area and operating the slider with a finger and the comment is inputted by inputting in the field or by selecting one of the predefined texts.

13. The acupuncture and moxibustion treatment support terminal device according to any one of Claims 1 to 12,
wherein the display input unit displays an interview sheet, and, by filling in items of the interview sheet, the items in the interview sheet are inputted in and displayed on the display input unit and are stored in the acupuncture and moxibustion treatment support terminal device.

14. The acupuncture and moxibustion treatment support terminal device according to Claim 13,
wherein, in a case in which one of the items of the interview sheet is to be inputted, one of the plurality of predefined texts or one of the plurality of displayed buttons is selected to input the item.

15. The acupuncture and moxibustion treatment support terminal device according to any one of Claims 1 to 14,
wherein the display input unit displays an image of a product and a button for submitting a purchase order of the product.

16. The acupuncture and moxibustion treatment support terminal device according to any one of Claims 1 to 15,
wherein the acupuncture and moxibustion treatment support terminal device further comprises; a reservation reception unit for accepting a reservation of a patient,
wherein the acupuncture and moxibustion treatment support terminal device stores electronic medical charts on a plurality of patients and a reservation calendar in which appointments of the patients accepted by the reservation reception unit are inputted, and
wherein, by means of the display input unit, the reservation calendar is displayed, and the appointments of the patients accepted by the reservation reception unit are inputted in the reservation calendar.

17. The acupuncture and moxibustion treatment support terminal device according to Claim 16,
wherein the acupuncture and moxibustion treatment support terminal device accepts a reservation of a new patient who has not yet been registered, assigns a specific time zone as a reserved time from an available time zone in the reservation calendar, accepts an input of information on the electronic medical charts on the new patient, and causes the display input unit to display the reserved time and the information on the electronic medical charts in association with each other.

18. A medical support system in which at least one clinic that performs a complementary and alternative medicine, a medical center at which a doctor is enrolled, and a home of a patient to receive the complementary and alternative medicine are connected to a server via a network,
wherein the clinic is provided with the acupuncture and moxibustion treatment support terminal device according to any one of claims 1 to 17, the medical center is provided with a personal computer or the acupuncture and moxibustion treatment support terminal device according to any one of claims 1 to 17, the acupuncture and moxibustion treatment support terminal device at the clinic and the personal computer or the acupuncture and moxibustion treatment support terminal device at the medical center exchange the information on the electronic medical charts of the patient with each other via the network, and the patient makes a reservation with the clinic via the network.

19. The medical support system according to Claim 18,
wherein the at least one clinic that performs the complementary and alternative medicine includes a plurality of clinics, and acupuncturists at respective clinics exchange with one another information including the information on the electronic medical charts on the patient via the network.

20. The medical support system according to Claim 18 or Claim 19,
wherein the information on the electronic medical charts on the patient exchanged via the network is outputted to existing electronic medical charts stored in a medical institution.

21. The medical support system according to any one of Claims 18 to 20,
wherein the acupuncture and moxibustion treatment support terminal device provided at the clinic displays an advertisement for the patient's reference via the network or a screen for the patient to purchase a product.
